(19) Europäisches Patentamt
European Patent Office
Office européen des brevets

(11) Veröffentlichungsnummer: **0 116 518 B1**

(12) **EUROPÄISCHE PATENTSCHRIFT**

(45) Veröffentlichungstag der Patentschrift: **28.10.87**

(21) Anmeldenummer: **84810056.6**

(22) Anmeldetag: **30.01.84**

(51) Int. Cl.⁴: **C 07 D 251/16,** C 07 D 239/46, C 07 D 403/12, C 07 D 417/12, C 07 D 401/12, A 01 N 47/36 // C07C143/80, C07D207/40, C07D207/38, C07D275/02, C07D239/42

(54) **N-Phenylsulfonyl-N'-pyrimidinyl- und -triazinylharnstoffe.**

(30) Priorität: **04.02.83 CH 643/83**

(43) Veröffentlichungstag der Anmeldung: **22.08.84 Patentblatt 84/34**

(45) Bekanntmachung des Hinweises auf die Patenterteilung: **28.10.87 Patentblatt 87/44**

(84) Benannte Vertragsstaaten: **BE CH DE FR GB IT LI NL**

(56) Entgegenhaltungen:
**EP-A-0 001 515**
**EP-A-0 044 212**
**US-A-3 849 110**

**Die Akte enthält technische Angaben, die nach dem Eingang der Anmeldung eingereicht wurden und die nicht in dieser Patentschrift enthalten sind.**

(73) Patentinhaber: **CIBA-GEIGY AG, Klybeckstrasse 141, CH-4002 Basel (CH)**

(72) Erfinder: **Schurter, Rolf, Dr., Holzmattstrasse 45, CH-4102 Binningen (CH)**
Erfinder: **Thummel, Rudolph C., Route d'Alle 424, CH-2892 Courgenay (CH)**
Erfinder: **Töpfl, Werner, Dr., Dorneckstrasse 68, CH-4143 Dornach (CH)**
Erfinder: **Meyer, Willy, Talweg 49, CH-4125 Riehen (CH)**
Erfinder: **Dürr, Dieter, Dr., Brändelistalweg 16, CH-4103 Bottmingen (CH)**

EP 0 116 518 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft neue, herbizid wirksame und pflanzenwuchsregulierende N-Phenylsulfonyl-N'-pyrimidinyl- und -triazinylharnstoffe, Verfahren zu ihrer Herstellung, sie als Wirkstoffe enthaltende Mittel, sowie deren Verwendung zum Bekämpfen von Unkräutern, vor allem selektiv in Nutzpflanzenkulturen oder zum Regulieren und Hemmen des Pflanzenwachstums. Darüber hinaus betrifft die Erfindung auch als Zwischenprodukte hergestellte neue Phenylsulfonamide.

Die erfindungsgemässen N-Phenylsulfonyl-N'-pyrimidinyl- und -triazinyl-harnstoffe entsprechen der allgemeinen Formel I

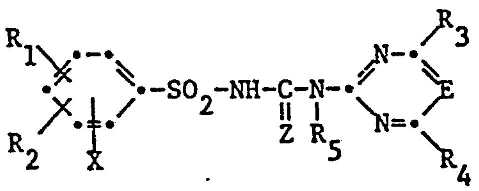 (I),

worin

E Stickstoff -N= oder die Methingruppe -CH=,

$R_1$ Wasserstoff, Halogen, Nitro, $C_1$–$C_5$ Alkyl, $C_1$–$C_5$ Halogenalkyl, $C_1$–$C_5$ Alkoxy, $C_1$–$C_5$ Halogenalkoxy oder $C_1$–$C_5$ Alkoxycarbonyl,

$R_2$ Wasserstoff,

$R_3$ $C_1$–$C_3$ Alkyl, $C_1$–$C_3$ Halogenalkyl, $C_1$–$C_3$ Alkoxy oder $C_1$–$C_3$ Halogenalkoxy,

$R_4$ Wasserstoff, Halogen, dasselbe wie $R_3$, $C_1$–$C_4$ Alkyl- oder Dialkylamino,

$R_5$ Wasserstoff oder Methyl,

X eine Gruppe $-NR_6R_7$ oder 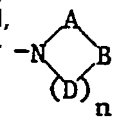

$R_6$ $C_3$–$C_6$ Alkenyl, $C_3$–$C_6$ Alkinyl, $C_3$–$C_7$ Cycloalkyl, $C_5$–$C_7$ Cycloalkenyl, unsubstituiert oder $C_1$–$C_6$ Alkyl, $C_3$–$C_6$ Alkenyl, $C_3$–$C_6$ Alkinyl, $C_3$–$C_7$ Cycloalkyl, substituiert durch Halogen, Cyan, $-COC_1$–$C_5$ Alkyl, $COC_1$–$C_5$ Halogenalkyl, -COOH, $-COO$-$C_1$–$C_5$ Alkyl, $COO$-$C_1$–$C_5$ Halogenalkyl, $-COC_2$–$C_{10}$ Alkoxyalkyl, $CO$-$COOC_1$–$C_5$ Alkyl oder $R_6$ ist eine Gruppe $COC_1$–$C_5$ Halogenalkyl, $-COC_2$–$C_{10}$ Alkoxyalkyl, $COC_3$–$C_7$ Cycloalkyl oder $-COCOOC_1$–$C_5$ Alkyl, wobei $R_6$ auch $-SO_2CH_3$ bedeutet, wenn $R_7$ Wasserstoff ist,

$R_7$ ist Wasserstoff, $C_1$–$C_4$ Alkyl, dasselbe wie $R_6$ oder $-SO_2\,C_1$–$C_4$ Alkyl,

A eine Gruppe -CO-, $-SO_2$-, -COO-, -CONH- oder $-CONC_1$–$C_5$ Alkyl-,

B $C_1$–$C_4$ Alkylen oder $C_2$–$C_4$ Alkenylen,

D eine Gruppe -CO-, $-SO_2$-, $-CHC_1$–$C_5$ Alkyl oder $C(C_1$–$C_5$ Alkyl$)_2$,

n ist 0 oder 1,

E Stickstoff =N- oder Methin =CH- und

Z Sauerstoff bedeuten, sowie die Salze dieser Verbindungen.

Harnstoffverbindungen, Triazinverbindungen und Pyrimidinverbindungen mit herbizider Wirkung sind allgemein bekannt. Arylsulfamoylheterocyclyl-aminocarbamoylverbindungen mit herbizider und pflanzenwuchsregulierender Wirkung sind beispielsweise im niederländischen Patent Nr. 121 788 oder in der europäischen Patentpublikation Nr. 44 210 beschrieben. Ferner sind in den EP-A 1515 und 44 212 strukturähnliche Phenylsulfonylharnstoffe beschrieben.

In den Definitionen ist unter Alkyl geradkettiges oder verzweigtes Alkyl zu verstehen, z.B.: Methyl, Ethyl, n-Propyl, i-Propyl, die vier isomeren Butyl, n-Amyl, i-Amyl, 2-Amyl, 3-Amyl, n-Hexyl, i-Hexyl, oder die verschiedenen Heptyl-, Octyl- oder Nonylisomeren.

Unter Alkoxy ist zu verstehen: Methoxy, Ethoxy, n-Propyloxy, i-Propyloxy und die vier isomeren Butyloxyreste, insbesondere aber Methoxy, Ethoxy oder i-Propoxy.

Beispiele für Alkylthio sind Methylthio, Ethylthio, n-Propylthio, i-Propylthio und n-Butylthio, insbesondere aber Methylthio und Ethylthio.

Beispiele für Alkenylreste sind Allyl, Isopropenyl, 1-Propenyl, 1-Butenyl, 2-Butenyl, 3-Butenyl, 1-Isobutenyl, 2-Isobutenyl, 1-Pentenyl, 2-Pentenyl, 3-Pentenyl und 4-Pentenyl, insbesondere aber Allyl und 4-Pentenyl.

Unter Halogen in den Definitionen sowie in Halogenalkyl, -alkoxy, und -alkylthio sind Fluor, Chlor und Brom, vorzugsweise jedoch Fluor und Chlor zu verstehen.

Alkinylresten in den Definitionen der obigen Symbole entsprechen in der Regel Propargyl, 2-Butinyl, 3-Butinyl sowie isomere Pentinyl- oder Hexinylreste, vorzugsweise ist der Alkinylrest jedoch durch Propargyl oder 2- oder 3-Butinyl verkörpert.

Die Erfindung umfasst ebenfalls die Salze, die die Verbindungen der Formel I mit Aminen, Alkali- und Erdalkalimetallbasen oder quaternären Ammoniumbasen bilden können.

Unter Alkali- und Erdalkalimetallhydroxiden als Salzbildner sind die Hydroxide von Lithium, Natrium, Kalium, Magnesium oder Calcium hervorzuheben, insbesondere aber die von Natrium oder Kalium.

Beispiele für zur Salzbildung geeignete Amine sind primäre, sekundäre und tertiäre aliphatische und aromatische Amine wie Methylamin, Ethylamin, Propylamin, i-Propylamin, die vier isomere Butylamine, Dimethylamin, Diethylamin, Diethanolamin, Dipropylamin, Diisopropylamin, Di-n-butylamin, Pyrrolidin, Piperidin, Morpholin, Trimethylamin, Triethylamin, Tripropylamin, Chinuclidin, Pyridin, Chinolin und i-Chinolin, insbesondere aber Ethyl-, Propyl-, Diethyl- oder Triethylamin, vor allem aber iso-Propylamin und Diethanolamin.

Beispiele für quaternäre Ammoniumbasen sind im allgemeinen die Kationen von Halogenammoniumsalzen, z.B. das Tetramethylammoniumkation, das Trimethylbenzylammoniumkation, das Triethylbenzylammoniumkation, das Tetraethylammoniumkation, das Trimethylethylammoniumkation, aber auch das Ammoniumkation.

Von den erfindungsgemässen Verbindungen der Formel sind die Verbindungen bevorzugt, in

denen

$R_1$ Wasserstoff, Halogen oder $C_1$–$C_5$ Alkoxycarbonyl,

$R_2$ Wasserstoff,

$R_3$ $C_1$–$C_3$ Alkyl, $C_1$–$C_3$ Halogenalkyl, $C_1$–$C_3$ Alkoxy oder $C_1$–$C_3$ Halogenalkoxy,

$R_4$ Wasserstoff, Halogen, dasselbe wie $R_3$ oder Di$C_1$–$C_3$ Alkylamin,

$R_5$ Wasserstoff oder Methyl,

X $NHC_2$–$C_5$ Alkenyl, $NHC_3$–$C_7$ Cycloalkyl oder $NHC_5$–$C_7$ Cycloalkenyl, welche unsubstituiert oder durch Halogen, Cyano, $COC_1$–$C_5$ Halogenalkyl, $COC_2$–$C_6$ Alkoxyalkyl oder $COOC_1$–$C_5$ Alkyl substituiert sind; X ist ferner NHCHO, $NHCOC_1$–$C_5$ Halogenalkyl, $NHCOOC_1$–$C_5$ Halogenalkyl, $NHCONHC_1$–$C_5$ Halogenalkyl, $NHCOCOOC_1$–$C_5$ Alkyl, $NHSO_2C_1$–$C_5$ Alkyl, $N(SO_2C_1$–$C_5$ Alkyl$)_2$, oder X ist ein über Stickstoff gebundener 5–6gliedriger gesättigter Heterocyclus, der noch ein Sauerstoff-, Schwefel- oder Stickstoffatom im Ring enthalten kann und mindestens eine Oxo-Gruppe enthalten muss.

Als bevorzugte Einzelverbindungen sind zu nennen:

N-(2-Trifluoracetamidophenylsulfonyl)-N′-(4-methoxy-6-methyl-1,3,5-triazin-2-yl)-harnstoff,
N-[2-(2′,2′-Dichlorpropionylamido)phenylsulfonyl]-N′-(4-methoxy-6-methyl-1,3,5-triazin-2-yl)-harnstoff,
N-(2-Chloracetylamidophenylsulfonyl)-N′-(4-methoxy-6-methyl-1,3,5-triazin-2-yl)-harnstoff,
N-(2-Methylsulfonamidophenylsulfonyl)-N′-(4-methoxy-6-methyl-1,3,5-triazin-2-yl)-harnstoff,
N-(2-Bromacetamidophenylsulfonyl)-N′-(4-methoxy-6-methyl-1,3,5-triazin-2-yl)-harnstoff,
N-(2-Dichloracetamidophenylsulfonyl)-N′-(4-methoxy-6-methyl-1,3,5-triazin-2-yl)-harnstoff,
N-(2-Methoxyacetamidophenylsulfonyl)-N′-(4-methoxy-6-methyl-1,3,5-triazin-2-yl)-harnstoff,
N-[2-Chloracetylamido-6-chlorphenylsulfonyl]-N′-(4-methoxy-6-methyl-1,3,5-triazin-2-yl)-harnstoff,
N-[2-(4′-Chlorbutyrylamido)phenylsulfonyl]-N′-(4-methoxy-6-methyl-1,3,5-triazin-2-yl)-harnstoff,
N-(2-Methoxyoxalylamidophenylsulfonyl)-N′-(4-methoxy-6-methyl-1,3,5-triazin-2-yl)-harnstoff,
N-(2-Ethoxyoxalylamidophenylsulfonyl)-N′-(4-methoxy-6-methyl-1,3,5-triazin-2-yl)-harnstoff,
N-[2-(4′-Chlorbutyrylamido)phenylsulfonyl]-N′-(4,6-dimethoxy-1,3,5-triazin-2-yl)-harnstoff,
N-[2-(4′-Chlorbutyrylamido)-6-chlorphenylsulfonyl]-N′-(4-methoxy-6-methyl-1,3,5-triazin-2-yl)-harnstoff,
N-[2-(2′-Piperidonyl)phenylsulfonyl]-N′-(4-methoxy-6-methyl-1,3,5-triazin-2-yl)-harnstoff,
N-(2-Trifluoracetamido-6-chlorphenylsulfonyl)-N′-(4-methoxy-6-methyl-1,3,5-triazin-2-yl)-harnstoff,
N-(2-Bromacetamido-6-chlorphenylsulfonyl)-N′-(4-methoxy-6-methyl-1,3,5-triazin-2-yl)-harnstoff,
N-(2-Trifluoracetamidophenylsulfonyl)-N′-(4-methoxy-6-methyl-pyrimidin-2-yl)-harnstoff,
N-(2-Bromacetamido-6-chlorphenylsulfonyl)-N′-(6-methoxy-6-methyl-pyrimidin-2-yl)-harnstoff,
N-(2-Chloracetamidophenylsulfonyl)-N′-(4-methoxy-6-methyl-pyrimidin-2-yl)-harnstoff,
N-[2-(4′-Chlorbutyrylamido)phenylsulfonyl]-N′-(4-methoxy-6-methyl-pyrimidin-2-yl)-harnstoff,
N-[2-(2′-Pyrrolidonyl)phenylsulfonyl]-N′-(4-methoxy-6-methyl-pyrimidin-2-yl)-harnstoff,
N-[2-(4′-Chlorbutyrylamido)-6-chlor-phenylsulfonyl]-N′-(4-methoxy-6-methyl-pyridimin-2-yl)-harnstoff,
N-[2-(2′-Piperidonyl)-phenylsulfonyl]-N′-(4-methoxy-6-methyl-pyrimidin-2-yl)-harnstoff.

Die Herstellung der Verbindungen der Formel I erfolgt in einem inerten, organischen Lösungsmittel.

Nach einem ersten Verfahren werden die Verbindungen der Formel I erhalten, indem man ein Phenylsulfonamid der Formel II

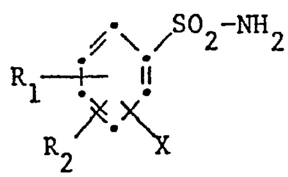

(II),

worin X, $R_1$ und $R_2$ die unter Formel I gegebene Bedeutung haben, in Gegenwart einer Base mit einem N-Pyrimidinyl- oder -Triazinylcarbamat der Formel III umsetzt,

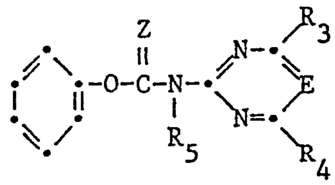

(III)

worin E, $R_3$, $R_4$, $R_5$ und Z die unter Formel I gegebene Bedeutung haben und der Phenylrest gegebenenfalls substituiert sein kann.

In einem zweiten Verfahren gelangt man zu Verbindungen der Formel I, indem man ein Phenylsulfonylisocyanat oder -isothiocyanat der Formel IV

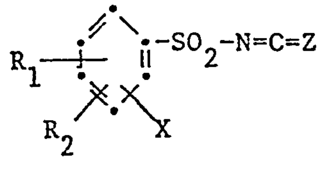

(IV),

worin X, $R_1$, $R_2$ und Z die unter Formel I gegebene Bedeutung haben, gegebenenfalls in Gegenwart einer Base, mit einem Amin der Formel V

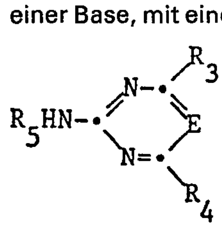

(V),

worin R, $R_3$, $R_4$ und $R_5$ die unter Formel I gegebene Bedeutung haben, umsetzt.

Nach einem weiteren Verfahren werden die Verbindungen der Formel I hergestellt, in denen $R_5$ Wasserstoff bedeutet, indem man ein Sulfonamid der oben angegebenen Formel II gegebenenfalls in Gegenwart einer Base mit einem Isocyanat oder Isothiocyyanat der Formel VI

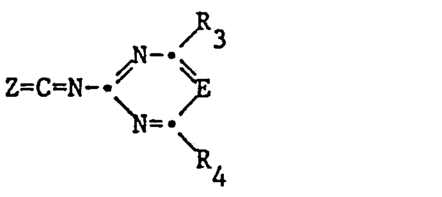 (VI),

worin E, $R_3$, $R_4$ und Z die unter Formel I gegebene Bedeutung haben, umsetzt.

Schliesslich kann man die Verbindungen der Formel I auch erhalten, indem man ein N-Phenylsulfonylcarbamat der Formel VII

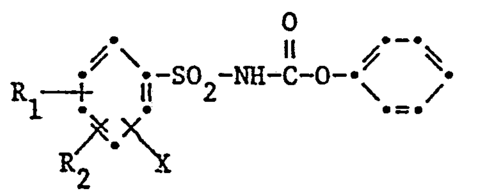 (VII)

worin x, $R_1$, $R_2$ und m die unter Formel I gegebene Bedeutung haben und der Phenoxyrest gegebenenfalls substituiert sein kann, mit einem Amin der oben angegebenen Formel V umsetzt.

Die erhaltenen Harnstoffe der Formel I können gewünschtenfalls mittels Aminen, Alkalimetall- oder Erdalkalimetallhydroxiden oder quaternären Ammoniumbasen in Additionssalze übergeführt werden. Dieses geschieht beispielsweise durch Umsetzen mit der äquimolaren Menge Base und Verdampfen des Lösungsmittels.

Die als Zwischenprodukte verwendeten Verbindungen der Formel II sind neu und speziell für die Synthese von Verbindungen der Formel I entwickelt worden. Diese Zwischenprodukte bilden einen weiteren Aspekt der vorliegenden Erfindung.

Die Ausgangsstoffe zur Herstellung der Verbindungen der Formeln II, III, V und VI sind bekannt oder können nach bekannten Methoden hergestellt werden.

Die Phenylsulfonylisocyanate der Formel IV können durch Umsetzungen der Phenylsulfonamide der Formel II mit Phosgen in Anwesenheit von Butylisocyanat in einem chlorierten Kohlenwasserstoff als Lösungsmittel, bei Rückflusstemperatur erhalten werden. Ähnliche Darstellungen sind in «Neuere Methoden der präparativen organischen Chemie», Band VI, 211–229, Verlag Chemie, Weinheim, 1970, beschrieben.

Die Isothiocyanate der Formel IV werden durch Behandlung der Phenylsulfonamide der Formel II mit Schwefelkohlenstoff und Kaliumhydroxid und anschliessender Umsetzung des Dikaliumsalzes mit Phosgen erhalten. Solche Verfahren sind in Arch. Pharm. 299, 174 (1966) beschrieben.

Die N-Phenylsulfonylcarbamate der Formel VII werden durch Umsetzung der Phenylsulfonamide der Formel II mit Diphenylcarbonat in Gegenwart einer Base erhalten. Ähnliche Verfahren sind in der japanischen Patentschrift 61 169 erwähnt.

Durch Umsetzung von heterocyclischen Aminen der Formel V mit Oxalylchlorid in chlorierten Kohlenwasserstoffen als Lösungsmittel, lassen sich Isocyanate der Formel VI herstellen, Amine der Formel V sind bekannt und zum Teil im Handel erhältlich, oder sie können nach bekannten Methoden hergestellt werden, siehe «The Chemistry of Heterocyclic Compounds», Band XIV, Interscience Publishers, New York, London.

Die Umsetzungen zu Verbindungen der Formel I werden vorteilhafterweise in aprotischen, inerten, organischen Lösungsmitteln vorgenommen wie Methylenchlorid, Tetrahydrofuran, Acetonitril, Dioxan, Toluol.

Die Reaktionstemperaturen liegen vorzugsweise zwischen -20° und +120°C. Die Umsetzungen verlaufen im allgemeinen leicht exotherm und können bei Raumtemperatur durchgeführt werden. Zwecks Abkürzung der Reaktionszeit oder auch zum Einleiten der Umsetzung wird zweckdienlich für kurze Zeit bis zum Siedepunkt des Reaktionsgemisches aufgewärmt. Die Reaktionszeiten können ebenfalls durch Zugabe von Base als Reaktionskatalysator verkürzt werden.

Die Wirkstoffe der Formel I sind stabile Verbindungen. Ihre Handhabung bedarf keiner vorsorglicher Massnahmen.

Bei geringeren Aufwandmengen zeichnen sich die Verbindungen der Formel I durch gute selektiv-wuchshemmende und selektiv-herbizide Eigenschaften aus, die sie ausgezeichnet zum Einsatz in Kulturen von Nutzpflanzen, insbesondere in Getreide, Baumwolle, Soja, Mais und Reis, befähigen. Es werden dabei teilweise auch Unkräuter geschädigt, welchen bisher nur mit Totalherbiziden beizukommen war.

Die Wirkungsart dieser Wirkstoffe ist unüblich. Viele sind translozierbar, d.h., sie werden von der Pflanze aufgenommen und an andere Stellen transportiert, wo sie dann zur Wirkung kommen. So gelingt es beispielsweise, durch Oberflächenbehandlung perenierende Unkräuter bis in die Wurzeln zu schädigen. Die neuen Verbindungen der Formel I wirken bereits bei – im Vergleich zu anderen Herbiziden und Wuchsregulatoren – sehr geringen Aufwandmengen.

Die Verbindungen der Formel I haben ausserdem starke pflanzenwuchsregulierende, insbesondere pflanzenwuchshemmende, Eigenschaften. Es werden sowohl Monokotyledonen als auch Dikotyledonen in ihrem Wachstum beeinträchtigt.

So können z.B. die in der Landwirtschaft in tropischen Gegenden häufig als «cover crops» (Bodenbedecker) angepflanzten Leguminosen durch die Verbindungen der Formel I in ihrem Wachstum selektiv gehemmt werden, so dass zwar die

Bodenerosion zwischen den Kulturpflanzen verhindert wird, die «cover crops» jedoch nicht zur Konkurrenz für die Kultur werden können.

Weiter eignen sich die Verbindungen der Formel I um das Keimen von eingelagerten Kartoffeln zu verhindern. Bei Kartoffeln entwickeln sich bei der Einlagerung über den Winter häufig Keime, die Schrumpfen, Gewichtsverlust und Faulen zur Folge haben.

Bei grösseren Aufwandmengen werden alle getesteten Pflanzen in ihrer Entwicklung so geschädigt, dass sie absterben.

Die Erfindung betrifft auch herbizide und pflanzenwachstumsregulierende Mittel, welche einen neuen Wirkstoff der Formel I enthalten, sowie Verfahren zur pre- und post-emergenten Unkrautbekämpfung und zur Hemmung des Pflanzenwuchses von monokotylen und dikotylen Pflanzen, insbesondere Gräsern, tropischen Bodenbedeckern und Tabakgeiztrieben.

Die Verbindungen der Formel I werden in unveränderter Form oder vorzugsweise als Mittel zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnissen entsprechend gewählt.

Die Formulierungen, d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwaserstoffe, bevorzugt die Fraktionen $C_8$ bis $C_{12}$, wie z.B. Xylolgemische oder substituierte Naphthaline, Phthalsäureester wie Dibutyl- oder Dicotylphthalat, aliphatische Kohlenwasserstoffe wie Cyclohexan oder Paraffine, Alkohole und Glykole sowie deren Ether und Ester, wie Ethanol, Ethylenglykol, Ethylenglykolmonomethyl- oder -ethylether, Ketone wie Cyclohexanon, stark polare Lösungsmittel, wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüber hinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen seien die Alkali-, Erdalkali- oder gegebenenfalls substituierte Ammoniumsalze von höheren Fettsäuren ($C_{10}$–$C_{22}$), wie z.B. die Na- oder K-Salze der Öl- oder Stearinsäure, oder von natürlichen Fettsäuregemischen, die z.B. aus Kokosnuss- oder Talgöl gewonnen werden können, genannt. Ferner sind auch die Fettsäure-methyltaurin-salze zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Ethylenoxid-Addukten. Die sulfonierten Benzimidazolderivate enthalten vorzugsweise 2 Sulfonsäuregruppen und einen Fettsäurerest mit 8–22 C-Atomen. Alkylarylsulfonate sind z.B. die Na-, Ca- oder Triethanolaminsalze der Dodecylbenzolsulfonsäure, der Dibutylnaphthalinsulfonsäure oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Ethylenoxid-Adduktes in Frage.

Als nicht ionische Tenside kommen in erster Linie Polyglykoletherderivate von aliphatischen oder cycloaliphatischen Alkoholen, gesättigten oder ungesättigten Fettsäuren und Alkylphenolen in Frage, die 3 bis 30 Glykolethergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Ethylenglykoläthergruppen und 10 bis 100 Propylenglykolethergruppen enthaltenden Polyethylenoxidaddukte an Polypropylenglykol, Ethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Ethylenglykoleinheiten.

Als Beispiele nicht ionischer Tenside seien Nonylphenolpolyethoxyethanole, Ricinusölpolyglykolether, Polypropylen-Polyethylenoxidaddukte, Tributylphenoxypolyethanol, Polyethylenglykol und Octylphenoxypolyethoxyethanol erwähnt.

Ferner kommen auch Fettsäureester von Polyoxyäthylensorbitan wie das Polyoxyäthylensorbitan-trioleat in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl oder niedrige Hydroxyalkylreste aufweisen. Die Salze liegen vorzugsweise als Halogenide, Methylsulfate oder Ethylsulfate vor, z.B. das Stearyltrimethylammoniumchlorid oder das Benzyldi(2-chlorethyl)-ethylammoniumbromid.

Die in der Formulierungstechnik gebräuchlichen Tenside sind u.a. in folgenden Publikationen beschrieben:

«Mc Cutcheon's Detergents and Emulsifiers Annual» MC Publishing Corp., Ridgewood, New Jersey, 1979. J. and M. Ash, «Encyclopedia of Surfactants» Vol. I-III Chemical Publishing Co., Inc. New York, 1980/81. M. Stache «Tensid-Taschenbuch» 2. Auflage, C. Hanser Verlag München und Wien, 1981.

Die herbiziden Zubereitungen enthalten in der Regel 0,1 bis 95%, insbesondere 0,1 bis 80%, Wirkstoff der Formel I, 1 bis 99,9% eines festen oder flüssigen Zusatzstoffes und 0 bis 25%, insbesondere 0,1 bis 25% eines Tensides.

Insbesondere setzen sich bevorzugte Formulierungen folgendermassen zusammen: (% = Gewichtsprozent)

Emulgierbare Konzentrate
Aktiver Wirkstoff:
1 bis 20%, bevorzugt 5 bis 10%
oberflächenaktives Mittel:
5 bis 30%, vorzugsweise 10 bis 20%
flüssiges Trägermittel:
50 bis 94%, vorzugsweise 70 bis 85%.

Stäube
Aktiver Wirkstoff:
0,1 bis 10%, vorzugsweise 0,1 bis 1%
festes Trägermittel:
99,9 bis 90%, vorzugsweise 99,9 bis 99%.

Suspension-Konzentrate
Aktiver Wirkstoff:
5 bis 75%, vorzugsweise 10 bis 50%
Wasser:
94 bis 25%, vorzugsweise 90 bis 30%
oberflächenaktives Mittel:
1 bis 40%, vorzugsweise 2 bis 30%.

Benetzbare Pulver
Aktiver Wirkstoff:
0,5 bis 90%, vorzugsweise 1 bis 80%
oberflächenaktives Mittel:
0,5 bis 20%, vorzugsweise 1 bis 15%
festes Trägermittel:
5 bis 95%, vorzugsweise 15 bis 90%.

Granulate
Aktiver Wirkstoff:
0,5 bis 30%, vorzugsweise 3 bis 15%
festes Trägermittel:
99,5 bis 70%, vorzugsweise 97 bis 85%.

Während als Handelsware eher konzentrierte Mittel bevorzugt werden, verwendet der Endverbraucher in der Regel verdünnte Mittel. Die Anwendungsformen können bis hinab zu 0,001% an Wirkstoff verdünnt werden. Die Aufwandmengen betragen in der Regel 0,01 bis 10 kg AS/ha, vorzugsweise 0,025 bis 5 kg As/ha.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

In den folgenden Beispielen sind die Temperaturen in Celsiusgraden °C angegeben.

Beispiel 1

Herstellung von N-[2-(4′-Chlorbutyrylamido)phenylsulfonyl]-N′-(4-methoxy-6-methyl-1,3,5-triazin-2-yl)-harnstoff

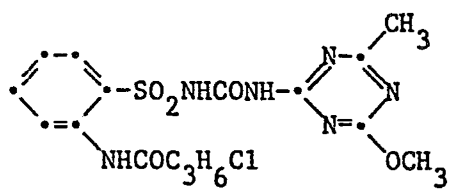

a) 2-(4′-Chlorubutyrylamido)benzolsulfonamid

Zu einer Lösung von 17,2 g (0,1 Mol) 2-Aminobenzolsulfonamid, 200 ml Acetonitril und 8,9 ml Pyridin tropft man während 2 Stunden bei Raumtemperatur und unter Rühren eine Lösung von 15,5 g (0,11 Mol) 4-Chlorbuttersäurechlorid in 50 ml Acetonitril. Nachdem alles zugetropft ist, rührt man 4 Stunden weiter, giesst dann die Reaktionslösung auf 2N Salzsäure-Eis-Gemisch und extrahiert zweimal mit Essigsäureethylester. Die organische Phase wird mit gesättigter Natriumbicarbonat- und mit gesättigter Natriumchloridlösung gewaschen, getrocknet, mit Aktivkohle behandelt und eingedampft. Der Rückstand wird aus Essigsäureethylester/Hexan umkristallisiert und ergibt 17,7 g (64% der Theorie) 2-(4-Chlorbutyrylamido)-benzolsulfonamid vom Schmelzpunkt 127–129°.

b) N-[2-(4′-Chlorbutyrylamido)phenylsulfonyl]-N′-(4-methoxy-6-methyl-1,3,5-triazin-2-yl)-harnstoff

Zu einer Lösung von 2,76 g (0,01 Mol) 2-(4′-Chlorbutyrylamido)-benzolsulfonamid in 30 ml

Dioxan werden 3,3 ml 1,8-Diazabicyclo[5.4.0]undec-7-en und 2,60 g (0,01 Mol) N-(4-Methoxy-6-methyl-1,3,5-triazin-2-yl)-phenylcarbamat gegeben und 24 Stunden bei 20-25° gerührt. Dann wird das Reaktionsgemisch auf 2N Salzsäure/Eis gegossen und während 2 Stunden gut gerührt. Der ausgefallene Niederschlag wird abfiltriert, mit 1N Salzsäure und Wasser gewaschen, getrocknet und aus Ethanol umkristallisiert. Man erhält so 2,6 g (59% der Theorie) des obigen Harnstoffes, welcher bei 144–145° schmilzt.

Analog zu diesem Beispiel werden die folgenden Verbindungen und Zwischenprodukte hergestellt.

Tabelle 1

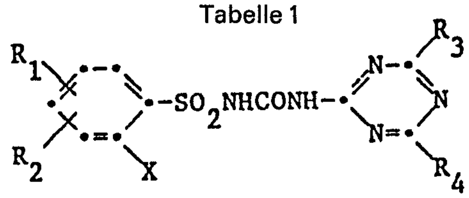

| No. | X | $R_1$ | $R_2$ | $R_3$ | $R_4$ | phys. Daten |
|---|---|---|---|---|---|---|
| 1.01 | $NHCOCF_3$ | H | H | $CH_3$ | $OCH_3$ | Smp. 198° Z |
| 1.02 | $NHCOCCl_2CH_3$ | H | H | $CH_3$ | $OCH_3$ | Smp. 140-143° |
| 1.03 | $NHCOCH_2Cl$ | H | H | $CH_3$ | $OCH_3$ | Smp. 165° Z |
| 1.04 | $NHSO_2CH_3$ | H | H | $CH_3$ | $OCH_3$ | Smp. 154-155° |
| 1.05 | $NHCOCH_2Br$ | H | H | $CH_3$ | $OCH_3$ | Smp. 151-152° Z |
| 1.06 | $NHCOCHCl_2$ | H | H | $CH_3$ | $OCH_3$ | Smp. 143° Z |
| 1.07 | $NHCOCH_2OCH_3$ | H | H | $CH_3$ | $OCH_3$ | Smp. 171-172° |
| 1.08 | 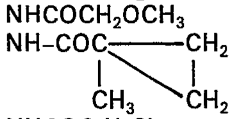 | H | H | $CH_3$ | $OCH_3$ | Smp. 146-149° |
| 1.09 | $NHCOC_3H_6Cl$ | H | H | $CH_3$ | $OCH_3$ | Smp. 144-145° |
| 1.10 | $NHCOCOOH_3$ | H | H | $CH_3$ | $OCH_3$ | Smp. 151° Z |
| 1.11 | $NHCOCOOC_2H_5$ | 6-Cl | H | $CH_3$ | $OCH_3$ | |
| 1.12 | $NHCOCH_2OCH_3$ | 6-Cl | H | $CH_3$ | $OCH_3$ | |
| 1.13 | $NHCOCH_2Cl$ | 6-Cl | H | $CH_3$ | $OCH_3$ | |
| 1.14 | 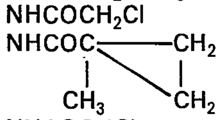 | 6-Cl | H | $CH_3$ | $OCH_3$ | |
| 1.15 | $NHCOCHCl_2$ | 6-Cl | H | $CH_3$ | $OCH_3$ | |
| 1.16 | $NHCOCF_3$ | 6-Cl | H | $CH_3$ | $OCH_3$ | |
| 1.17 | $NHCOCH_2Br$ | 6-Cl | H | $CH_3$ | $OCH_3$ | |
| 1.18 | $NHSO_2CH_3$ | 6-Cl | H | $CH_3$ | $OCH_3$ | Smp. 139° Z |
| 1.19 | $NHCOCCl_2CH_3$ | 6-Cl | H | $CH_3$ | $OCH_3$ | |
| 1.20 | $NHCOCH_2Cl$ | H | H | $OCH_3$ | $OCH_3$ | |
| 1.21 | $NHCOCH_2Cl$ | H | H | $OCH_3$ | $N(CH_3)_2$ | |
| 1.22 | $NHCOCH_2Cl$ | 6-Cl | H | $OCH_3$ | $OCH_3$ | |
| 1.23 | $NHCOCH_2Cl$ | 6-Cl | H | $OCH_3$ | $N(CH_3)_2$ | |
| 1.24 | $NHCOCH_2Br$ | H | H | $OCH_3$ | $OCH_3$ | |
| 1.25 | $NHCOCH_2Br$ | 6-Cl | H | $OCH_3$ | $OCH_3$ | |
| 1.26 | $NHCOCH_2Cl$ | $6\text{-}COOCH_3$ | H | $CH_3$ | $OCH_3$ | |
| 1.27 | $NHCOCH_2OCH_3$ | $6\text{-}COOCH_3$ | H | $CH_3$ | $OCH_3$ | |
| 1.28 | NHCHO | H | H | $CH_3$ | $OCH_3$ | Smp. 156° Z |
| 1.29 | N(Cyclopropyl)$SO_2CH_3$ | H | H | $CH_3$ | $OCH_3$ | Smp. 178-180° |
| 1.30 | 2-Pyrrolidon-1-yl | H | H | $CH_3$ | $OCH_3$ | |
| 1.31 | 2-Pyrrolidon-1-yl | 6-Cl | H | $CH_3$ | $OCH_3$ | |
| 1.32 | 2-Pyrrolidon-1-yl | 6-Cl | H | $OCH_3$ | $N(CH_3)_2$ | |
| 1.33 | 1,1-Dioxo-2-isothiazolidinyl | H | H | $CH_3$ | $OCH_3$ | Smp. 212° Z |
| 1.34 | 1,1-Dioxo-2-isothiazolidinyl | 6-Cl | H | $CH_3$ | $OCH_3$ | |
| 1.35 | 2-Pyrrolidon-1-yl | $6\text{-}COOCH_3$ | H | $CH_3$ | $OCH_3$ | |
| 1.36 | 2-Piperidon-1-yl | H | H | $CH_3$ | $OCH_3$ | |
| 1.37 | 1,3-Oxazol-2-on-3-yl | H | H | $CH_3$ | $OCH_3$ | |
| 1.38 | $-N(CH_3)CH_2CH{=}CH_2$ | H | H | $CH_3$ | $OCH_3$ | |
| 1.39 | 2,5-Pyrrolidindion-1-yl | H | H | $CH_3$ | $OCH_3$ | Smp. 202-203° Z |
| 1.40 | 2,5-Pyrrolidindion-1-yl | 6-Cl | H | $CH_3$ | $OCH_3$ | |
| 1.41 | $NHCOCOOC_2H_5$ | H | H | $CH_3$ | $OCH_3$ | Smp. 119° Z |
| 1.42 | $NHCH{=}C(COOC_2H_5)_2$ | H | H | $CH_3$ | $OCH_3$ | Smp. 173-176° |
| 1.43 | $NHCOCOOCH_3$ | 6-Cl | H | $CH_3$ | $OCH_3$ | Smp. 147° Z |

Tabelle 2

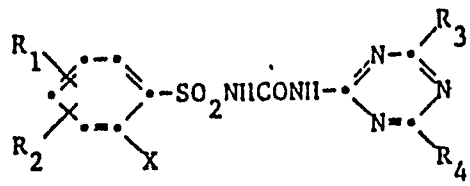

| No. | X | $R_1$ | $R_2$ | $R_3$ | $R_4$ | phys. Daten |
|---|---|---|---|---|---|---|
| 2.01 | $NH-CH=C(COOC_2H_5)_2$ | H | H | $CH_3$ | $OCH_3$ | Smp. 182–184° Z |
| 2.02 | $NH-CH=C(CN)COOC_2H_5$ | H | H | $CH_3$ | $OCH_3$ | |
| 2.03 | $NH-CH=C(COOCH_3)_2$ | H | H | $CH_3$ | $OCH_3$ | |
| 2.04 | $NH-C(CH_3)=CHCOCH_3$ | H | H | $CH_3$ | $OCH_3$ | Smp. 163–166° Z |
| 2.05 | $NH-C(CH_3)=CHCOCF_3$ | H | H | $CH_3$ | $OCH_3$ | Smp. 184–186° Z |
| 2.06 | $NH-C(CH_3)=CH-COOCH_3$ | H | H | $CH_3$ | $OCH_3$ | Smp. 157–159° Z |
| 2.07 | 2-Methoxycarbonylcyclopent-1-enylamino | H | H | $CH_3$ | $OCH_3$ | Smp. 155–157° Z |
| 2.08 | NHCHO | H | H | $CH_3$ | $OCH_3$ | Smp. 176° Z |
| 2.09 | $NHCOC_3H_6Cl$ | H | H | $CH_3$ | $OCH_3$ | Smp. 112 Z |
| 2.10 | 2-Pyrrolidon-1-yl | H | H | $CH_3$ | $OCH_3$ | |
| 2.11 | 1,1-Dioxo-2-isothiazolidinyl | H | H | $CH_3$ | $OCH_3$ | Smp. 204° Z |
| 2.12 | 2-Piperidinon-1-yl | H | H | $CH_3$ | $OCH_3$ | Smp. 181–182° Z |
| 2.13 | $N(SO_2CH_3)_2$ | H | H | $CH_3$ | $OCH_3$ | Smp. 185–190° Z |
| 2.14 | 2-Pyrrolidon-1-yl | 6-Cl | H | $CH_3$ | $OCH_3$ | Smp. 150° Z |
| 2.15 | $NHCOCF_3$ | H | H | $CH_3$ | $OCH_3$ | Smp. 180–188° Z |

Tabelle 3

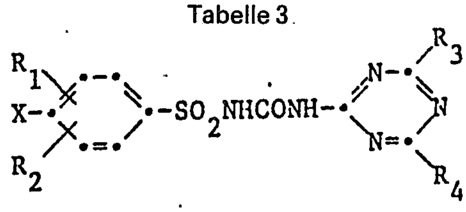

| No. | X | $R_1$ | $R_2$ | $R_3$ | $R_4$ | phys. Daten |
|---|---|---|---|---|---|---|
| 3.01 | $NHCH=C(COOC_2H_5)_2$ | H | H | $CH_3$ | $OCH_3$ | |
| 3.02 | $NHCH=C(CN)COOC_2H_5$ | H | H | $CH_3$ | $OCH_3$ | |
| 3.03 | $NHC(CH_3)=CHCOCH_3$ | H | H | $CH_3$ | $OCH_3$ | |
| 3.04 | $NHC(CH_3)=CHCOCF_3$ | H | H | $CH_3$ | $OCH_3$ | |
| 3.05 | $NHC(CH_3)=CHCOOCH_3$ | H | H | $CH_3$ | $OCH_3$ | |
| 3.06 | 2-Methoxycarbonyl-cyclopent-1-enylamino | H | H | $CH_3$ | $OCH_3$ | |
| 3.07 | NHCHO | H | H | $CH_3$ | $OCH_3$ | |
| 3.08 | $NHCOC_3H_6Cl$ | H | H | $CH_3$ | $OCH_3$ | Smp. 185° Z |
| 3.09 | Pyrrolidon-1-yl | H | H | $CH_3$ | $OCH_3$ | Smp. 168° Z |

Tabelle 4

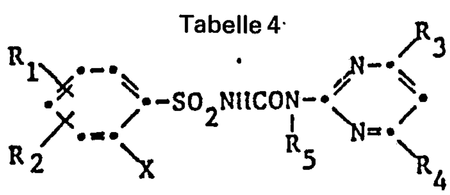

| No. | X | $R_1$ | $R_2$ | $R_3$ | $R_4$ | $R_5$ | phys. Daten |
|---|---|---|---|---|---|---|---|
| 4.01 | $NHCOCF_3$ | H | H | $CH_3$ | $OCH_3$ | H | Smp.156–158° Z |
| 4.02 | $NHCOCH_2OCH_3$ | H | H | $CH_3$ | $OCH_3$ | H | Smp. 167° Z |
| 4.03 | $NHCOCH_2Cl$ | H | H | $CH_3$ | $OCH_3$ | H | Smp. 124–125° Z |
| 4.04 | $NHCOCCl_2CH_3$ | H | H | $CH_3$ | $OCH_3$ | H | |
| 4.05 | $NHSO_2CH_3$ | H | H | $CH_3$ | $OCH_3$ | H | |

Tabelle 4 (Fortsetzung)

| No. | X | $R_1$ | $R_2$ | $R_3$ | $R_4$ | $R_5$ | phys. Daten |
|---|---|---|---|---|---|---|---|
| 4.06 | $NHCOC_3H_6Cl$ | H | H | $CH_3$ | $OCH_3$ | H | Smp. 130–132° Z |
| 4.07 | $NHCOCOOCH_3$ | H | H | $CH_3$ | $OCH_3$ | H | |
| 4.08 | $NHCOF_3$ | 6-Cl | H | $CH_3$ | $OCH_3$ | H | |
| 4.09 | $NHCOCF_3$ | 6-Cl | H | $CH_3$ | $OCHF_2$ | H | |
| 4.10 | $NHCOCF_3$ | 6-$COOCH_3$ | H | $CH_3$ | $OCH_3$ | H | |
| 4.11 | $NHCOCH_2OCH_3$ | 6-Cl | H | $CH_3$ | $OCH_3$ | H | |
| 4.12 | $NHCOCH_2OCH_3$ | 6-Cl | H | $CH_3$ | $OCHF_2$ | H | |
| 4.13 | $NHCOCH_2OCH_3$ | 6-$COOCH_3$ | H | $CH_3$ | $OCH_3$ | H | |
| 4.14 | $NHCOCH_2OCH_3$ | 6-Cl | H | $OCH_3$ | $OCH_3$ | H | |
| 4.15 | $NHCOCH_2Cl$ | 6-Cl | H | $OCH_3$ | $OCH_3$ | H | |
| 4.16 | $NHCOCH_2Cl$ | 6-Cl | H | $CH_3$ | $OCH_3$ | H | |
| 4.17 | $NHCOCH_2Cl$ | 6-$COOCH_3$ | H | $CH_3$ | $OCH_3$ | H | |
| 4.18 | $NHCOCH_2Cl$ | 6-$COOCH_3$ | H | $CH_3$ | $OCHF_2$ | H | |
| 4.19 | $NHCOCH_2Cl$ | 6-$COOCH_3$ | H | $CH_3$ | $OCH_3$ | H | |
| 4.20 | $NHCOCCl_2CH_3$ | 6-Cl | H | $CH_3$ | $OCH_3$ | H | |
| 4.21 | $NHSO_2CH_3$ | 6-Cl | H | $CH_3$ | $OCH_3$ | H | |
| 4.22 | $NHCOC_3H_6C_1$ | 6-Cl | H | $CH_3$ | $OCH_3$ | H | Smp. 124° Z |
| 4.23 | $NHCOC_3H_6Cl$ | 6-Cl | H | $OCH_3$ | $OCH_3$ | H | |
| 4.24 | $NHCOC_3H_6Cl$ | 6-Cl | H | $CH_3$ | $OCHF_3$ | H | Smp. 138° |
| 4.25 | $NHCOC_3H_6Cl$ | 6-$COOCH_3$ | H | $CH_3$ | $OCH_3$ | H | |
| 4.26 | $NHCOC_3H_6Cl$ | 6-$COOCH_3$ | H | $CH_3$ | $OCHF_2$ | H | |
| 4.27 | $NHCOCOOCH_3$ | 6-Cl | H | $CH_3$ | $OCH_3$ | H | Smp. 164° Z |
| 4.28 | $NHCOCOOCH_3$ | 6-Cl | H | $CH_3$ | $OCH_3$ | $CH_3$ | |
| 4.29 | $NHCOCOOCH_3$ | 6-Cl | H | $OCHF_2$ | $OCHF_2$ | H | |
| 4.30 | $NH-CH=C(COOC_2H_5)_2$ | H | H | $CH_3$ | $Cl_3$ | H | Smp. 197–200° Z |
| 4.31 | 2-Pyrrolidon-1-yl | H | H | $CH_3$ | $OCH_3$ | H | Smp. 126–128° Z |
| 4.32 | 2-Pyrrolidon-1-yl | 6-Cl | H | $CH_3$ | $OCH_3$ | H | |
| 4.33 | 2-Pyrrolidon-1-yl | 6-Cl | H | $CH_3$ | $OCHF_2$ | H | |
| 4.34 | 1,1-Dioxo-2-isothiazo-lidinyl | H | H | $CH_3$ | $OCH_3$ | H | |
| 4.35 | 1,1-Dioxo-2-isothiazo-lidinyl | 6-Cl | H | $CH_3$ | $OCH_3$ | H | |
| 4.36 | 1,1-Dioxo-2-isothiazo-lidinyl | 6-Cl | H | $CH_3$ | $OCHF_2$ | H | |
| 4.37 | 2-Pyrrolidon-1-yl | 6-$COOCH_3$ | H | $CH_3$ | $OCH_3$ | H | |
| 4.38 | 2-Piperidon-1-yl | H | H | $CH_3$ | $OCH_3$ | H | |
| 4.39 | 1,3-Diazolidin-2-on-1-yl | H | H | $CH_3$ | $OCH_3$ | H | |
| 4.40 | 3-Methyl-1,3-diazoli-din-2-on-1-yl | H | H | $CH_3$ | $OCH_3$ | H | |
| 4.41 | 1,3-Diazolidin-2,5-dion-1-yl | H | H | $CH_3$ | $OCH_3$ | H | |
| 4.42 | 1,3-Oxazolidin-2-on-3-yl | H | H | $CH_3$ | $OCH_3$ | H | |
| 4.43 | 5-Methyl-1,3-oxazoli-din-2,4-dion-3-yl | H | H | $CH_3$ | $OCH_3$ | H | |
| 4.44 | $NH(CH_3)CH_2CH=CH_2$ | H | H | $CH_3$ | $OCH_3$ | H | |
| 4.45 | 2,5-Pyrrolidindion-1-yl | H | H | $CH_3$ | $OCH_3$ | H | Smp. 193–195° Z |
| 4.46 | 2,5-Pyrrolidindion-1-yl | 6-Cl | H | $CH_3$ | $OCH_3$ | H | |
| 4.47 | 2,5-Pyrrolidindion-1-yl | H | H | $CH_3$ | $CH_3$ | H | Smp. 185° Z |
| 4.48 | $NH-CH=C(COOC_2H_3)_2$ | H | H | $CH_3$ | $OCH_3$ | H | Smp. 182–184° |
| 4.49 | 2-Pyrrolidon-1-yl | H | H | $OCH_3$ | $OCH_3$ | H | |
| 4.50 | 2-Pyrrolidon-1-yl | 6-Cl | H | $OCH_3$ | $OCH_3$ | H | |
| 4.51 | $NH(CH_3)COCH_3$ | H | H | $CH_3$ | $OCH_3$ | H | |
| 4.52 | $NH(CH_3)COCH_3$ | H | H | $OCH_3$ | $OCH_3$ | H | |
| 4.53 | $NH(CH_3)COCH_3$ | 6-Cl | H | $OCH_3$ | $OCH_3$ | H | |

Tabelle 5

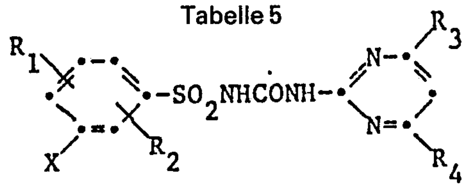

| No. | X | $R_1$ | $R_2$ | $R_3$ | $R_4$ | phys. Daten |
|---|---|---|---|---|---|---|
| 5.01 | $NHCH=C(COOC_2H_5)_2$ | H | H | $CH_3$ | $OCH_3$ | Smp. 182–188° |
| 5.02 | $NHCH=C(CN)COOC_2H_5$ | H | H | $CH_3$ | $OCH_3$ | Smp. 180–195° Z |
| 5.03 | $NHCH=C(COOCH_3)_2$ | H | H | $CH_3$ | $OCH_3$ | |
| 5.04 | $NHC(CH_3)=CHCOCH_3$ | H | H | $CH_3$ | $OCH_3$ | Smp. 171–173° Z |
| 5.05 | $NHC(CH_3)=CHCOCF_3$ | H | H | $CH_3$ | $OCH_3$ | Smp. 211–213° Z |
| 5.06 | $NHC(CH_3)=CHCOOCH_3$ | H | H | $CH_3$ | $OCH_3$ | Smp. 154–158° Z |
| 5.07 | 2-Methoxycarbonyl-cyclopent-1-enylamino | H | H | $CH_3$ | $OCH_3$ | Smp. 167–170° Z |
| 5.08 | NHCHO | H | H | $CH_3$ | $OCH_3$ | |
| 5.09 | $NHCOC_3H_6Cl$ | H | H | $CH_3$ | $OCH_3$ | Smp. 155° Z |
| 5.10 | 2-Pyrrolidon-1-yl | H | H | $CH_3$ | $OCH_3$ | Smp. 235° Z |
| 5.11 | 1,1-Dioxo-isothiazolidinyl | H | H | $CH_3$ | $OCH_3$ | Smp. 197–198° Z |
| 5.12 | 2-Piperidon-1-yl | H | H | $CH_3$ | $OCH_3$ | Smp. 133–136° Z |
| 5.13 | $N(SO_2CH_3)_2$ | H | H | $CH_3$ | $CH_3$ | Smp. 160° Z |
| 5.14 | $N(SO_2CH_3)_2$ | H | H | $CH_3$ | $OCH_3$ | Smp. 221–223° Z |
| 5.15 | 2-Pyrrolidon-1-yl | 6-Cl | H | $CH_3$ | $OCH_3$ | Smp. 195–196° Z |
| 5.16 | $NHCH=C(CN)COOC_2H_5$ | H | H | $CH_3$ | $CH_3$ | Smp. 195–196° Z |
| 5.17 | 1-Cyano-1-cyclohexylamino | H | H | $CH_3$ | $OCH_3$ | Smp. 162–165° Z |
| 5.18 | $NHC(CH_3)=CHCOCF_3$ | H | H | $CH_3$ | $CH_3$ | Smp. 199–201° Z |

Tabelle 6

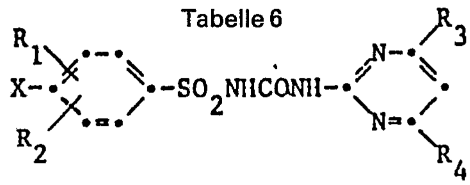

| No. | X | $R_1$ | $R_2$ | $R_3$ | $R_4$ | phys. Daten |
|---|---|---|---|---|---|---|
| 6.01 | $NHCH=C(COOC_2H_5)_2$ | H | H | $CH_3$ | $OCH_3$ | Smp. 200–203° Z |
| 6.02 | $NHCH=C(CN)COOC_2H_5$ | H | H | $CH_3$ | $OCH_3$ | |
| 6.03 | $NHC(CH_3)=CHCOCH_3$ | H | H | $CH_3$ | $OCH_3$ | |
| 6.04 | $NHC(CH_3)=CHCOCF_3$ | H | H | $CH_3$ | $OCH_3$ | |
| 6.05 | $NHC(CH_3)=CHCOOCH_3$ | H | H | $CH_3$ | $OCH_3$ | |
| 6.06 | 2-Methoxycarbonylcyclohexen-1-ylamino | H | H | $CH_3$ | $OCH_3$ | |
| 6.07 | NHCHO | H | H | $CH_3$ | $OCH_3$ | |
| 6.08 | $NHCOC_3H_6Cl$ | H | H | $CH_3$ | $OCH_3$ | Smp. 193° Z |
| 6.09 | 2-Pyrrolidon-1-yl | H | H | $CH_3$ | $OCH_3$ | |
| 6.10 | $N(SO_2CH_3)_2$ | H | H | $CH_3$ | $OCH_3$ | Smp. 236–238° Z |
| 6.11 | $NHC(CH_3)_2CN$ | H | H | $CH_3$ | $CH_3$ | Smp. 164–166° Z |
| 6.12 | $NHCH_2CN$ | H | H | $CH_3$ | $OCH_3$ | Smp. 198–200° Z |

Tabelle 7: Zwischenprodukte

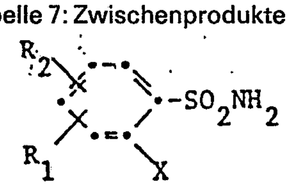

| No. | X | $R_1$ | $R_2$ | phys. Daten |
|---|---|---|---|---|
| 7.01 | $NHCOF_3$ | H | H | |
| 7.02 | $NHCOCCl_2CH_3$ | H | H | |

Tabelle 7 (Fortsetzung)

| No. | X | $R_1$ | $R_2$ | phys. Daten |
|---|---|---|---|---|
| 7.03 | $NHCOCH_2Cl$ | H | H | |
| 7.04 | $NHSO_2CH_3$ | H | H | |
| 7.05 | $NHCOCH_2Br$ | H | H | |
| 7.06 | $NHCOCHCl_2$ | H | H | |
| 7.07 | $NHCOCH_2OCH_3$ | H | H | |
| 7.08 | $NHCOC_3H_6Cl$ | H | H | Smp. 127-129° |
| 7.09 | $NHCOCOOCH_3$ | H | H | Smp. 202-203° |
| 7.10 | $NHCOCOOC_2H_5$ | H | H | Smp. 187° |
| 7.11 | $NHCOCH_2OCH_3$ | 6-Cl | H | |
| 7.12 | $NHCOCH_2Cl$ | 6-Cl | H | Smp. 163-165° |
| 7.13 | $NHCOCF_3$ | 6-Cl | H | |
| 7.14 | $NHCOCH_2Br$ | 6-Cl | H | |
| 7.15 | $NHSO_2CH_3$ | 6-Cl | H | Smp. 178° |
| 7.16 | 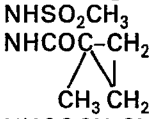 | 6-Cl | H | |
| 7.17 | $NHCOCH_2Cl$ | $6-COOCH_3$ | H | |
| 7.18 | $NHCOC_3H_6Cl$ | $6-COOCH_3$ | H | |
| 7.19 | $NHCOC_3H_6Cl$ | 6-Cl | H | Smp. 128-130° |
| 7.20 | NHCHO | H | H | |
| 7.21 | 1,1-Dioxo-2-isothiazolidinyl | H | H | |
| 7.22 | $N(CH_3)CH_2CH=CH_2$ | H | H | |
| 7.23 | $NHCOCOOCH_3$ | 6-Cl | H | Smp. 201-202° Z |
| 7.24 | 2,5-Pyrrolidindion-1-yl | H | H | Smp. 251° Z |
| 7.25 | 2,5-Pyrrolidindion-1-yl | 6-Cl | H | |

Tabelle 8: Zwischenprodukte

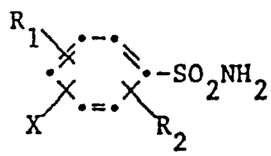

| No. | X | $R_1$ | $R_2$ | phys. Daten |
|---|---|---|---|---|
| 8.01 | $3-NHCOC_3H_6Cl$ | H | H | Smp. 131° |
| 8.02 | 3-(2-Pyrrolidon-1-yl | H | H | Smp. 198-199° |
| 8.03 | 3-(1,1-Dioxo-isothiazolidinyl) | H | H | Smp. 152° |
| 8.04 | 3-(2,5-Pyrrolidion-1-yl) | H | H | |
| 8.05 | 3-NHCHO | H | H | |
| 8.06 | $3-NHCH=C(COOC_2H_5)_2$ | H | H | Smp. 173-175° |
| 8.07 | $3-NHCH=C(CN)_2$ | H | H | |
| 8.08 | $3-NHC(CH_3)=CHCOCH_3$ | H | H | Smp. 159-161° |
| 8.09 | $3-NHC(CH_3)=CHCOCF_2$ | H | H | Smp. 149-151 |
| 8.10 | $3-NHC(CH_3)=CH-COOCH_3$ | H | H | Smp. 150-152° |
| 8.11 | 3-(2-Methoxycarbonyl-cyclo-hex-1-en-yl-amino) | H | H | Smp. 180-182° |
| 8.12 | $3-N(SO_2CH_3)_2$ | H | H | Smp. 203-206° |
| 8.13 | 3-(2-Piperidon-1-yl) | H | H | |
| 8.20 | $4-NHCOC_3H_6Cl$ | H | H | Smp. 183° |
| 8.21 | 4-(2-Pyrrolidon-1-yl) | H | H | Smp. 244-245° |
| 8.22 | 4-NHCHO | H | H | |
| 8.23 | $4-NHCH=C(COOC_2H_5)_2$ | H | H | Smp. 152-154 |
| 8.24 | $4-NHC(CH_3)=CHCOCH_3$ | H | H | |
| 8.25 | $4-NHC(CH_3)=CHCOCF_3$ | H | H | |
| 8.26 | $4-NHC(CH_3)=CHCOOCH_3$ | H | H | |
| 8.27 | 4-(2-Methoxycarbonyl-cyclo-hex-1-en-ylamino) | H | H | |

Tabelle 9: Zwischenprodukte

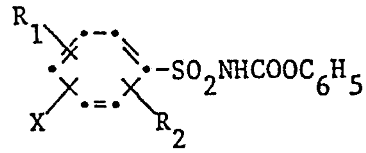

| No. | X | $R_1$ | $R_2$ | phys. Daten |
|---|---|---|---|---|
| 9.01 | 3-(2-Pyrrolidon-1-yl) | H | H | |
| 9.02 | 3-(1,1-Dioxo-isothiazolidinyl) | H | H | |
| 9.03 | 3-(2-Piperidon-1-yl) | H | H | |

Formulierungsbeispiele

Beispiel 2

Formulierungsbeispiele für Wirkstoffe der Formel I (% = Gewichtsprozent)

| a) Spritzpulver | a) | b) | c) |
|---|---|---|---|
| Wirkstoff | 20% | 60% | 0,5% |
| Na-Ligninsulfonat | 5% | 5% | 5% |
| Na-Laurylsulfat | 3% | – | – |
| Na-Diisobutylnaphthalin-sulfonat | – | 6% | 6% |
| Octylphenolpolyethylen-glykolether (7–8 Mol AeO) | – | 2% | 2% |
| Hochdisperse Kieselsäure | 5% | 27% | 27% |
| Kaolin | 67% | – | – |
| Natriumchlorid | – | – | 59,5% |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| b) Emulsion-Konzentrat | a) | b) |
|---|---|---|
| Wirkstoff | 10% | 1% |
| Octylphenolpolyethylen-glykolether (4–5 Mol AeO) | 3% | 3% |
| Ca-Dodecylbenzolsulfonat | 3% | 3% |
| Ricinusölpolyglykolether (36 Mol AeO) | 4% | 4% |
| Cyclohexanon | 30% | 10% |
| Xylolgemisch | 50% | 79% |

Aus diesem Konzentrat können durch Verdünnen mit Wasser Emulsionen jeder gewünschten Konzentration hergestellt werden.

| c) Stäubemittel | a) | b) |
|---|---|---|
| Wirkstoff | 0,1% | 1% |
| Talkum | 99,9% | – |
| Kaolin | – | 99% |

Man erhält anwendungsfertige Stäubemittel, indem der Wirkstoff mit dem Träger vermischt und auf einer geeigneten Mühle vermahlen wird.

| d) Extruder Granulat | a) | b) |
|---|---|---|
| Wirkstoff | 10% | 1% |
| Na-Ligninsulfonat | 2 | 2% |
| Carboxymethylcellulose | 1% | 1% |
| Kaolin | 87% | 96% |

Der Wirkstoff wird mit den Zusatzstoffen vermischt, vermahlen und mit Wasser angefeuchtet. Dieses Gemisch wird extrudiert und anschliessend im Luftstrom getrocknet.

| e) Umhüllungs-Granulat | |
|---|---|
| Wirkstoff | 3% |
| Polyethylenglykol (MG 200) | 3% |
| Kaolin | 94% |

Der fein gemahlene Wirkstoff wird in einem Mischer auf das mit Polyethylenglykol angefeuchtete Kaolin gleichmässig aufgetragen. Auf diese Weise erhält man staubfreie Umhüllungs-Granulate.

| f) Suspensions-Konzentrat | a) | b) |
|---|---|---|
| Wirkstoff | 40% | 5% |
| Ethylenglykol | 10% | 10% |
| Nonylphenolpolyethylen-glykolether (15 Mol AwO) | 6% | 1% |
| Na-Ligninsulfonat | 10% | 5% |
| Carboxymethylcellulose | 1% | 1% |
| 37%ige wässrige Formaldehyd-Lösung | 0,2% | 0,2% |
| Silikonöl in Form einer 75%igen wässrigen Emulsion | 0,8% | 0,8% |
| Wasser | 32% | 77% |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

| g) Salzlösung | |
|---|---|
| Wirkstoff | 5% |
| Isopropylamin | 1% |
| Octylphenolpolyethylen-glykolether (78 Mol AeO) | 3% |
| Wasser | 91% |

Biologische Beispiele

Beispiel 3

Herbizidwirkung vor dem Auflaufen der Pflanzen

Kunststofftöpfe werden mit expandiertem Vermiculit (Dichte: 0,135 g/cm³, Wasserabsorptionsvermögen: 0,565 l/l) gefüllt. Nach dem Sättigen des nicht adsorptiven Vermiculits mit einer wässrigen Wirkstoffemulsion in deionisiertem Wasser, die die Wirkstoffe in einer Konzentration von 70,8 ppm enthält, werden Samen der folgenden Pflanzen auf die Oberfläche gesät: Nasturium officinalis, Agrostis tenuis, Stellaria media und Digitaria sanguinalis. Die Versuchsgefässe werden anschliessend in einer Klimakammer bei 20%C, einer Beleuchtung von ca. 20 kLux und einer relativen Luftfeuchtigkeit von 70% gehalten. Während der Keimphase von 4 bis 5 Tagen werden die Töpfe zur Erhöhung der örtlichen Luftfeuchtigkeit mit lichtdurchlässigem Material abgedeckt und mit deionisiertem Wasser gegossen. Nach dem 5. Tag wird dem Giesswasser 0,5% eines handelsüblichen Flüssigdüngers (®Greenzit) zugesetzt. 12 Tage nach der Aussaat wird der Versuch ausgewertet und die Wirkung auf die Versuchspflanzen nach dem folgenden Massstab bewertet:

1 Pflanzen haben nicht gekeimt oder sind abgestorben
2–3 sehr starke Herbizidwirkung
4–6 mittlere Wirkung
7, 8 schwache Wirkung
9 keine Wirkung, Pflanzen gedeihen wie unbehandelte Kontrollen.

Die Resultate sind untenstehend zusammengefasst.

| Pflanze | Nasturtium officinalis | Agrostis tenuis | Stellaria media | Digitalis sanguinalis |
|---|---|---|---|---|
| Wirkstoff Nr. | | | | |
| 1.02 | 2 | 2 | 1 | 1 |
| 1.03 | 2 | 2 | 2 | 2 |
| 1.04 | 2 | 1 | 2 | 2 |
| 1.05 | 1 | 1 | 1 | 1 |
| 1.07 | 2 | 2 | 2 | 2 |
| 1.08 | 2 | 2 | 1 | 1 |
| 1.09 | 1 | 1 | 2 | 2 |
| 1.10 | 2 | 2 | 3 | 3 |
| 1.11 | 2 | 2 | 3 | 3 |
| 1.18 | 3 | 3 | 2 | 2 |
| 1.29 | 4 | 6 | 3 | 2 |
| 1.33 | 1 | 1 | 2 | 2 |
| 1.41 | 2 | 2 | 3 | 3 |
| 1.42 | 2 | 6 | 2 | 4 |
| 1.43 | 2 | 2 | 3 | 3 |
| 2.01 | 1 | 1 | 2 | 2 |
| 2.04 | 1 | 1 | 2 | 2 |
| 2.05 | 2 | 1 | 3 | 2 |
| 2.06 | 1 | 1 | 1 | 1 |
| 2.07 | 1 | 1 | 2 | 2 |
| 2.08 | 2 | 1 | 2 | 1 |
| 2.09 | 1 | 1 | 3 | 2 |
| 2.11 | 2 | 2 | 3 | 3 |
| 2.13 | 2 | 1 | 3 | 3 |
| 2.15 | 2 | 1 | 2 | 1 |
| 3.08 | 2 | 1 | 1 | 1 |
| 4.03 | 1 | 1 | 1 | 1 |
| 4.06 | 1 | 1 | 2 | 2 |
| 4.21 | 2 | 2 | 2 | 3 |
| 4.22 | 2 | 1 | 1 | 2 |
| 4.24 | 2 | 1 | 2 | 3 |
| 4.27 | 2 | 2 | 3 | 3 |
| 4.30 | 2 | 2 | 2 | 2 |
| 4.31 | 1 | 1 | 2 | 2 |
| 4.32 | 1 | 1 | 2 | 2 |
| 4.48 | 2 | 3 | 3 | 3 |
| 5.01 | 2 | 2 | 2 | 2 |
| 5.02 | 2 | 1 | 1 | 1 |
| 5.04 | 2 | 2 | 2 | 2 |
| 5.05 | 2 | 2 | 2 | 2 |

| Pflanze | Nasturtium officinalis | Agrostis tenuis | Stellaria media | Digitalis sanguinalis |
|---|---|---|---|---|
| 5.07 | 2 | 1 | 2 | 2 |
| 5.09 | 1 | 1 | 1 | 1 |
| 5.10 | 2 | 2 | 3 | 6 |
| 5.11 | 1 | 1 | 2 | 3 |
| 5.12 | 2 | 2 | 2 | 2 |
| 5.13 | 2 | 2 | 2 | 2 |
| 5.14 | 1 | 1 | 1 | 1 |
| 5.16 | 2 | 2 | 2 | 2 |
| 5.18 | 2 | 2 | 3 | 2 |
| 6.08 | 1 | 1 | 2 | 3 |
| 6.11 | 2 | 1 | 2 | 2 |

Beispiel 4

Nachweis der Keimhemmung an Lagerkartoffeln

Eine Anzahl im Handel erhältliche Kartoffeln der Sorte «Urgenta» ohne Keime werden gewaschen und abgetrockent. Danach werden die Kartoffeln für jeweils eine Minute in Wirkstoffemulsionen verschiedener Konzentration getaucht, in Kunststoffschalen auf Filterpapier ausgelegt und bei Temperaturen von 14° und 21°C im Dunkeln bei 50% relativer Luftfeuchtigkeit gehalten. Die Auswertung erfolgte 34 Tage nach der Applikation. Gleichzeitig wird der Gewichtsverlust der Knollen und das Gewicht der Keime im Vergleich zur unbehandelten Kontrolle ermittelt. Die erfindungsgemässen Verbindungen zeigten in diesem Versuch eine vollständige Verhinderung der Keimbildung. Gleichzeitig betrug der Gewichtsverlust der Kartoffeln weniger als 10% des Gewichtsverlustes der Kontrollkartoffeln.

Beispiel 5

Nachweis der Wuchshemmung bei tropischen Bodendeckern-Leguminosen (cover crops)

Die Versuchspflanzen (centrosema plumieri und centrosoma pubescens) werden bis zum ausgewachsenen Stadium herangezogen und bis auf eine Höhe von 60 cm zurückgeschnitten. Nach 7 Tagen wird der Wirkstoff als wässrige Emulsion gespritzt. Die Versuchspflanzen werden bei 70% relativer Luftfeuchtigkeit und 6000 lux Kunstlicht, pro Tag 14 Stunden, bei Temperaturen von 27°C bei Tag und 21°C bei Nacht gehalten. 4 Wochen nach der Applikation wird der Versuch ausgewertet. Es werden dabei der Neuzuwachs im Vergleich zur Kontrolle abgeschätzt und gewogen und die Phytotoxizität bewertet. In diesem Versuch zeigen die mit den Wirkstoffen der Formel I behandelten Pflanzen eine deutliche Reduktion des Neuzuwachses (weniger als 20% des Neuzuwachses bei unbehandelten Kontrollpflanzen), ohne dass die Versuchspflanzen geschädigt wurden.

**Patentansprüche**

1. N-Phenylsulfonyl-N'-pyrimidinyl- und -triazinyl-harnstoffe der Formel I

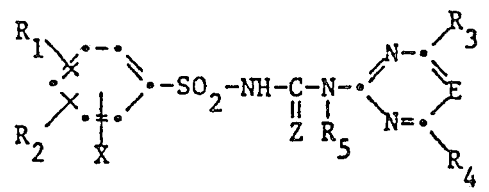

(I),

worin

E Stickstoff -N= oder die Methingruppe -CH=,

$R_1$ Wasserstoff, Halogen, Nitro, $C_1$-$C_5$ Alkyl, $C_1$-$C_5$ Halogenalkyl, $C_1$-$C_5$ Alkoxy, $C_1$-$C_5$ Halogenalkoxy oder $C_1$-$C_5$ Alkoxycarbonyl,

$R_2$ Wasserstoff,

$R_3$ $C_1$-$C_3$ Alkyl, $C_1$-$C_3$ Halogenalkyl, $C_1$-$C_3$ Alkoxy oder $C_1$-$C_3$ Halogenalkoxy,

$R_4$ Wasserstoff, Halogen, dasselbe wie $R_3$, $C_1$-$C_4$ Alkyl- oder Dialkylamino,

$R_5$ Wasserstoff oder Methyl,

X eine Gruppe $-NR_6R_7$ oder 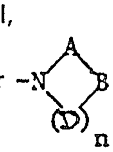

$R_6$ $C_3$-$C_6$ Alkenyl, $C_3$-$C_6$ Alkinyl, $C_3$-$C_7$ Cycloalkyl, $C_5$-$C_7$ Cycloalkenyl, unsubstituiert oder $C_1$-$C_6$ Alkyl, $C_3$-$C_6$ Alkenyl, $C_3$-$C_6$ Alkinyl, $C_3$-$C_7$ Cycloalkyl, substituiert durch Halogen, Cyan, $-COC_1$-$C_5$ Alkyl, $COC_1$-$C_5$ Halogenalkyl, -COOH, $-COO$-$C_1$-$C_5$ Alkyl, $COO$-$C_1$-$C_5$ Halogenalkyl, $-COC_2$-$C_{10}$ Alkoxyalkyl, $CO$-$COOC_1$-$C_5$ Alkyl oder $R_6$ ist eine Gruppe $COC_1$-$C_5$ Halogenalkyl, $-COC_2$-$C_{10}$ Alkoxyalkyl, $COC_3$-$C_7$ Cycloalkyl oder $-COCOOC_1$-$C_5$ Alkyl, wobei $R_6$ auch $-SO_2CH_3$ bedeutet, wenn $R_7$ Wasserstoff ist,

$R_7$ ist Wasserstoff, $C_1$-$C_4$ Alkyl, dasselbe wie $R_6$ oder $-SO_2$ $C_1$-$C_4$ Alkyl,

A eine Gruppe -CO-, $-SO_2$-, -COO-, -CONH- oder $-CONC_1$-$C_5$ Alkyl,

B $C_1$-$C_4$ Alkylen oder $C_2$-$C_4$ Alkenylen,

D eine Gruppe -CO-, $-SO_2$-, $-CHC_1$-$C_5$ Alkyl oder $C(C_1$-$C_5$ $Alkyl)_2$,

n ist 0 oder 1,

E Stickstoff =N- oder Methin =CH- und

Z Sauerstoff bedeuten, sowie die Salze dieser Verbindungen.

2. Die N-Phenylsulfonyl-N'-pyrimidinyl- oder -triazinyl-harnstoffe der Formel I gemäss Anspruch 1, worin
$R_1$ Wasserstoff, Halogen oder $C_1$-$C_5$ Alkoxycarbonyl,
$R_2$ Wasserstoff,
$R_3$ $C_1$-$C_3$ Alkyl, $C_1$-$C_3$ Halogenalkyl, $C_1$-$C_3$ Alkoxy oder $C_1$-$C_3$ Halogenalkoxy,
$R_4$ Wasserstoff, Halogen, dasselbe wie $R_3$ oder Di$C_1$-$C_3$Alkylamin,
$R_5$ Wasserstoff oder Methyl,
X $NHC_3$-$C_5$ Alkenyl, $NHC_3$-$C_7$ Cycloalkyl oder $NHC_5$-$C_7$ Cycloalkenyl, welche unsubstituiert oder durch Halogen, Cyano, $COC_1$-$C_5$ Halogenalkyl, $COC_2$-$C_6$ Alkoxyalkyl oder $COOC_1$-$C_5$ Alkyl substituiert sind; X ist ferner NHCHO, $NHCOC_1$-$C_5$ Halogenalkyl, $NHCOOC_1$-$C_5$ Halogenalkyl, $NHCONHC_1$-$C_5$ Halogenalkyl, $NHCOCOOC_1$-$C_5$ Alkyl, $NHSO_2C_1$-$C_5$ Alkyl, $N(SO_2C_1$-$C_5$ $Alkyl)_2$, oder X ist ein über Stickstoff gebundener 5-6gliedriger gesättigter Heterocyclus, der noch ein Sauerstoff-, Schwefel- oder Stickstoffatom im Ring enthalten kann und mindestens eine Oxo-Gruppe enthalten muss.

3. N-[2-(2',2'-Dichlorpropionylamido)phenylsulfonyl]-N'-(4-methoxy-6-methyl-1,3,5-triazin-2-yl)-harnstoff gemäss Anspruch 1.

4. N-(2-Chloracetamidophenylsulfonyl)-N'-(4-methoxy-6-methyl-1,3,5-triazin-2-yl)-harnstoff gemäss Anspruch 1.

5. N-(2-Methylsulfonylamido-6-chlorphenylsulfonyl)-N'-(4-methoxy-6-methyl-1,3,5-triazin-2-yl)-harnstoff gemäss Anspruch 1.

6. N-(2-Bromacetamidophenylsulfonyl)-N'-(4-methoxy-6-methyl-1,3,5-triazin-2-yl)-harnstoff gemäss Anspruch 1.

7. N-(2-Methoxyacetamidophenylsulfonyl)-N'-(4-methoxy-6-methyl-1,3,5-triazin-2-yl)-harnstoff gemäss Anspruch 1.

8. N-[2-Chloracetamido-6-chlorphenylsulfonyl]-N'-(4-methoxy-6-methyl-1,3,5-triazin-2-yl)-harnstoff gemäss Anspruch 1.

9. N-[2-(4'-Chlorbutyrylamido)phenylsulfonyl]-N'-(4-methoxy-6-methyl-1,3,5-triazin-2-yl)-harnstoff gemäss Anspruch 1.

10. N-(2-Methoxyoxalylamidophenylsulfonyl)-N'-(4-methoxy-6-methyl-1,3,5-triazin-2-yl)-harnstoff gemäss Anspruch 1.

11. N-(2-Methoxyoxalylamido-6-chlorphenylsulfonyl)-N'-(4-methoxy-6-methyl-1,3,5-triazin-2-yl)-harnstoff gemäss Anspruch 1.

12. N-(2-Chloracetamidophenylsulfonyl)-N'-(4-methoxy-6-methylpyrimidin-2-yl)-harnstoff gemäss Anspruch 1.

13. N-[2-(4'-Chlorbutyrylamido)phenylsulfonyl]-N'-(4-methoxy-6-methylpyrimidin-2-yl)-harnstoff gemäss Anspruch 1.

14. N-[2-(2'-Pyrrolidon-1'-yl)phenylsulfonyl]-N'-(4-methoxy-6-methylpyrimidin-2-yl)-harnstoff gemäss Anspruch 1.

15. Verfahren zur Herstellung der N-Phenylsulfonyl-N'-pyrimidinyl- und -triazinyl-harnstoffe der Formel I, Anspruch 1, dadurch gekennzeichnet, dass man ein Benzolsulfonamid der Formel II

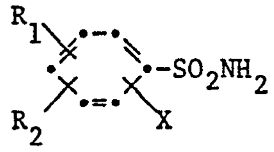

(II),

worin $R_1$, $R_2$ und X die unter Formel I gegebene Bedeutung haben, in Gegenwart einer Base mit einem N-Pyrimidinyl- oder -Triazinylcarbamat der Formel

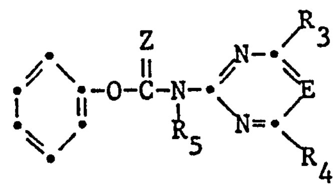

(III),

worin E, $R_3$, $R_4$, $R_5$ und Z die unter Formel I gegebene Bedeutung haben und der Phenylrest gegebenenfalls substituiert ist, umsetzt und gegebenenfalls in ihre Salze überführt.

16. Verfahren zur Herstellung der Verbindungen der Formel I, Anspruch 1, dadurch gekennzeichnet, dass man ein Phenylsulfonylisocyanat oder -isothiocyanat der Formel IV

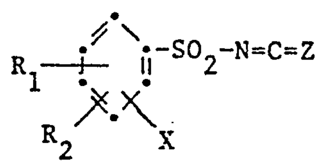

(IV),

worin $R_1$, $R_2$, X und Z die unter Formel I gegebene Bedeutung haben, gegebenenfalls in Gegenwart einer Base, mit einem Amin der Formel V

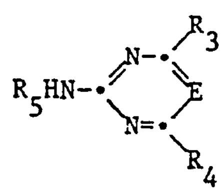

(V),

worin $R_3$, $R_4$ und $R_5$ die unter Formel I gegebene Bedeutung haben, umsetzt und gegebenenfalls in ihre Salze überführt.

17. Verfahren zur Herstellung der Verbindungen der Formel I, Anspruch 1, worin $R_5$ Wasserstoff bedeutet, dadurch gekennzeichnet, dass man Sulfonamide der im Anspruch 15 angegebenen Formel II gegebenenfalls in Gegenwart einer Base mit einem Isocyanat oder Isothiocyanat der Formel VI

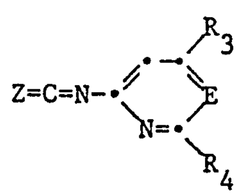

(VI)

worin E, $R_3$, $R_4$ und Z die unter Formel I gegebenen Bedeutungen haben, umsetzt und gegebenenfalls in ihre Salze überführt.

18. Verfahren zur Herstellung der Verbindungen der Formel I, Anspruch 1, dadurch gekennzeichnet, dass man ein N-Phenylsulfonylcarbamat der Formel VII

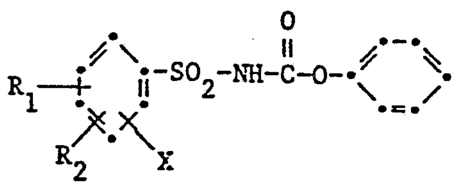 (VII)

worin $R_1$, $R_2$ und X die unter Formel I gegebene Bedeutung haben und der Phenylrest gegebenenfalls substituiert sein kann, mit einem Amin der oben angegebenen Formel V umsetzt und gegebenenfalls in ihre Salze überführt.

19. Verfahren zur Herstellung von Additionssalzen der Formel I, Anspruch 1, dadurch gekennzeichnet, dass man einen Sulfonylharnstoff der Formel I mit einem Amin, einem Alkalimetall- oder Erdalkalimetallhydroxid oder einer quaternären Ammoniumbase umsetzt.

20. Phenylsulfonamide der Formel II

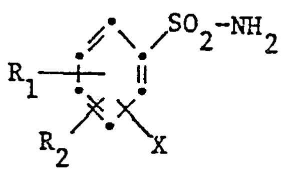 (II),

worin $R_1$, $R_2$ und X die unter Formel I, Anspruch 1, gegebene Bedeutung haben.

21. Ein herbizides und den Pflanzenwuchs hemmendes Mittel, dadurch gekennzeichnet, dass es neben Träger- und/oder anderen Zuschlagstoffen als Wirkstoff mindestens einen N-Phenylsulfonyl-N′-triazinyl- oder -pyrimidinylharnstoff der Formel I, Anspruch 1, enthält.

22. Die Verwendung der N-Phenylsulfonyl-N′-triazinyl- oder pyrimidinylharnstoffe der Formel I gemäss Anspruch 1 oder sie enthaltender Mittel zur selektiven pre- oder post-emergenten Bekämpfung von Unkräutern in Nutzpflanzenkulturen.

23. Die Verwendung der Verbindungen der Formel I gemäss Anspruch 1 oder sie enthaltender Mittel in Getreide-, Baumwolle-, Soja-, Mais- und Reiskulturen.

## Claims

1. N-phenylsulfonyl-N′-pyrimidinyl- and -N′-triazinylureas of the formula

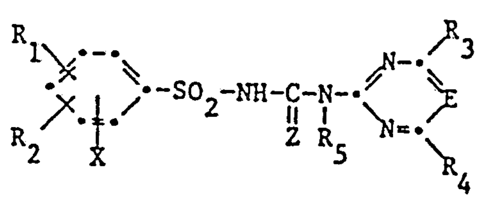 (I)

wherein

E is nitrogen -N= or the methine group -CH=,

$R_1$ is hydrogen, halogen, nitro, $C_1$–$C_5$alkyl, $C_1$–$C_5$haloalkyl, $C_1$–$C_5$alkoxy, $C_1$–$C_5$haloalkoxy or $C_1$–$C_5$alkoxycarbonyl,

$R_2$ is hydrogen,

$R_3$ is $C_1$–$C_3$alkyl, $C_1$–$C_3$haloalkyl, $C_1$–$C_3$alkoxy or $C_1$–$C_3$haloalkoxy,

$R_4$ is hydrogen, halogen or has the same meaning as $R_3$ or is $C_1$–$C_4$alkyl- or dialkyl-amino,

$R_5$ is hydrogen or methyl,

X is a $-NR_6R_7$ or 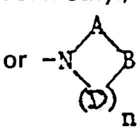

$R_6$ is $C_3$–$C_6$alkenyl, $C_3$–$C_6$alkynyl, $C_3$–$C_7$cycloalkyl, $C_5$–$C_7$cycloalkenyl, each unsubstituted, or $C_1$–$C_6$alkyl, $C_3$–$C_6$alkenyl, $C_3$–$C_6$alkynyl, $C_3$–$C_7$cycloalkyl, each substituted by halogen, cyano, $-COC_1$–$C_5$alkyl, $COC_1$–$C_5$haloalkyl, -COOH, $-COO-C_1$–$C_5$alkyl, $COO-C_1$–$C_5$haloalkyl, $-COC_2$–$C_{10}$alkoxyalkyl or by $CO$-$COOC_1$–$C_5$alkyl, or $R_6$ is a $COC_1$–$C_5$haloalkyl, $COC_2$–$C_{10}$alkoxyalkyl, $COC_3$–$C_7$cycloalkyl or $-COCOOC_1$–$C_5$alkyl group, $R_6$ also being $-SO_2CH_3$ when $R_7$ is hydrogen,

$R_7$ is hydrogen, $C_1$–$C_4$alkyl or has the same meaning as $R_6$ or is $-SO_2C_1$–$C_4$alkyl,

A is a -CO-, $-SO_2-$, -COO-, -CONH- or $-CONC_1$–$C_5$alkyl group,

B is $C_1$–$C_4$alkylene or $C_2$–$C_4$alkenylene,

D is a -CO-, $-SO_2-$, $-CHC_1$–$C_5$alkyl or $C(C_1$–$C_5$alkyl$)_2$ group,

n is 0 or 1,

E is nitrogen =N- or methine =CH-, and

Z is oxygen.

2. The N-phenylsulfonyl-N′-pyrimidinyl- and -N′-triazinyl-ureas of the formula I according to claim 1, wherein

$R_1$ is hydrogen, halogen or $C_1$–$C_5$alkoxycarbonyl,

$R_2$ is hydrogen,

$R_3$ is $C_1$–$C_3$alkyl, $C_1$–$C_3$haloalkyl, $C_1$–$C_3$alkoxy or $C_1$–$C_3$haloalkoxy,

$R_4$ is hydrogen, halogen or has the same meaning as $R_3$ or is di-$C_1$–$C_3$alkylamine,

$R_5$ is hydrogen or methyl,

X is $NHC_3$–$C_5$alkenyl, $NHC_3$–$C_7$cycloalkyl or $NHC_5$–$C_7$cycloalkenyl, each unsubstituted or substituted by halogen, cyano, $COC_1$–$C_5$haloalkyl, $COC_2$–$C_6$alkoxyalkyl or $COOC_1$–$C_5$alkyl; and X is also NHCHO, $NHCOC_1$–$C_5$haloalkyl, $NHCOOC_1$–$C_5$haloalkyl, $NHCONHC_1$–$C_5$haloalkyl, $NHCOCOOC_1$–$C_5$alkyl, $NHSO_2C_1$–$C_5$alkyl, $N(SO_2C_1$–$C_5$alkyl$)_2$; or X is a 5- or 6-membered saturated heterocycle which is bonded by way of nitrogen and may contain a further oxygen, sulfur or nitrogen atom in the ring and must contain at least one oxo group.

3. N-[2-(2′,2′-dichloropropionylamido)phenylsulfonyl]-N′-(4-methoxy-6-methyl-1,3,5-triazin-2-yl)urea according to claim 1.

4. N-(2-chloroacetamidophenylsulfonyl)-N′-(4-methoxy-6-methyl-1,3,5-triazin-2-yl)urea according to claim 1.

5. N-(2-methylsulfonylamido-6-chlorophenyl-

sulfonyl)-N′-(4-methoxy-6-methyl-1,3,5-triazin-2-yl)urea according to claim 1.

6. N-(2-bromoacetamidophenylsulfonyl)-N′-(4-methoxy-6-methyl-1,3,5-triazin-2-yl)urea according to claim 1.

7. N-(2-methoxyacetamidophenylsulfonyl)-N′-(4-methoxy-6-methyl-1,3,5-triazin-2-yl)urea according to claim 1.

8. N-[2-chloroacetamido-6-chlorophenylsulfonyl]-N′-(4-methoxy-6-methyl-1,3,5-triazin-2-yl)urea according to claim 1.

9. N-[2-(4′-chlorobutyrylamido)phenylsulfonyl]-N′-(4-methoxy-6-methyl-1,3,5-triazin-2-yl)urea according to claim 1.

10. N-(2-methoxyoxalylamidophenylsulfonyl)-N′-(4-methoxy-6-methyl-1,3,5-triazin-2-yl)urea according to claim 1.

11. N-(2-methoxyoxalylamido-6-chlorophenylsulfonyl)-N′-(4-methoxy-6-methyl-1,3,5-triazin-2-yl)urea according to claim 1.

12. N-(2-chloroacetamidophenylsulfonyl)-N′-(4-methoxy-6-methylpyrimidin-2-yl)urea according to claim 1.

13. N-[2-(4′-chlorobutyrylamido)phenylsulfonyl]-N′-(4-methoxy-6-methylpyrimidin-2-yl)urea according to claim 1.

14. N-[2-(2′-pyrrolidon-1′-yl)phenylsulfonyl]-N′-(4-methoxy-6-methylpyrimidin-2-yl)urea according to claim 1.

15. A process for the preparation of N-phenylsulfonyl-N′-pyrimidinyl- and -N′-triazinyl-ureas of the formula I, claim 1, characterised in that a benzenesulfonamide of the formula II

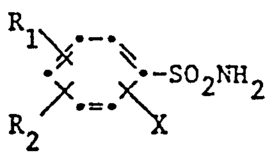 (II)

wherein $R_1$, $R_2$ and X are as defined for formula I, is reacted with an N-pyrimidinylcarbamate or N-triazinylcarbamate of the formula III

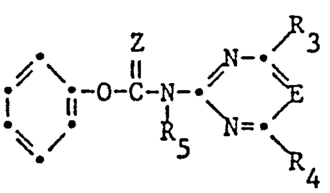 (III)

wherein E, $R_3$, $R_4$, $R_5$ and Z are as defined for formula I and the phenyl radical is unsubstituted or substituted, in the presence of a base, and, if desired, converted into salts thereof.

16. A process for the preparation of compounds of the formula I, claim 1, characterised in that a phenylsulfonylisocyanate or phenylsulfonylisothiocyanate of the formula IV

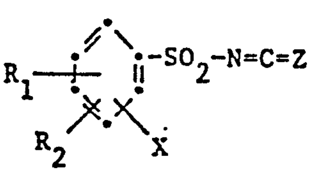 (IV),

wherein $R_1$, $R_2$, X and Z are as defined for formula I, is reacted with an amine of the formula V

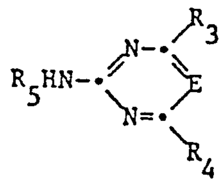 (V),

wherein $R_3$, $R_4$ and $R_5$ are as defined for formula I, optionally in the presence of a base, and, if desired, converted into salts thereof.

17. A process for the preparation of compounds of the formula I, claim 1, wherein $R_5$ is hydrogen, characterised in that sulfonamides of the formula II given in claim 15 are reacted with an isocyanate or isothiocyanate of the formula VI

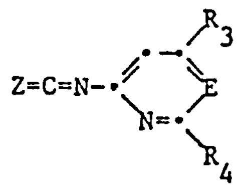 (VI),

wherein E, $R_3$, $R_4$ and Z are as defined for formula I, optionally in the presence of a base, and, if desired, converted into salts thereof.

18. A process for the preparation of compounds of the formula I, claim 1, characterised in that an N-phenylsulfonylcarbamate of the formula VII

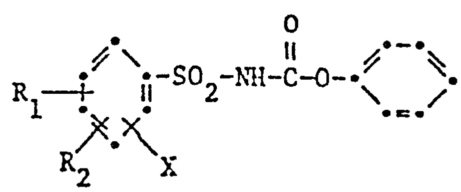 (VII),

wherein $R_1$, $R_2$ and X are as defined for formula I and the phenyl radical may be unsubstituted or substituted, is reacted with an amine of the formula V given above and, if desired, converted into salts thereof.

19. A process for the preparation of addition salts of the formula I, claim 1, characterised in that a sulfonylurea of the formula I is reacted with an amine, an alkali metal hydroxide or an alkaline earth metal hydroxide or with a quaternary ammonium base.

20. Phenylsulfonamides of the formula II

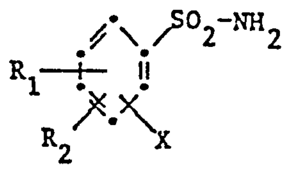 (II),

wherein $R_1$, $R_2$ and X are as defined for formula I, claim 1.

21. A herbicidal and plant growth inhibiting composition, characterised in that it contains as

active substance at least one N-phenylsulfonyl-N′-triazinyl- or -N′-pyrimidinyl-urea of the formula I, claim 1, together with carriers and/or other adjuvants.

22. The use of N-phenylsulfonyl-N′-triazinyl- or -N′-pyrimidinyl-ureas of the formula I according to claim 1, or of compositions containing them, for the selective control of weeds pre- and post-emergence in crops of useful plants.

23. The use of compounds of the formula I according to claim 1, or of compositions containing them, in crops of cereals, cotton, soybeans, maize and rice.

**Revendications**

1 N-phénylsulfonyl-N′-pyrimidinyl- et -triazinyl-urées de formule

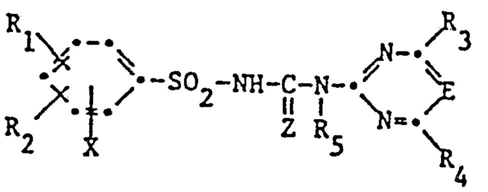 (I)

dans laquelle

E représente l'azote -N= ou le groupe méthine -CH=,

$R_1$ représente l'hydrogène, un halogène, un groupe nitro, alkyle en $C_1$–$C_5$, halogénoalkyle en $C_1$–$C_5$, alcoxy en $C_1$–$C_5$, halogénoalcoxy en $C_1$–$C_5$ ou alcoxycarbonyle en $C_1$–$C_5$,

$R_2$ représente l'hydrogène,

$R_3$ représente un groupe alkyle en $C_1$–$C_3$, halogénoalkyle en $C_1$–$C_3$, alcoxy en $C_1$–$C_3$ ou halogénoalcoxy en $C_1$–$C_3$,

$R_4$ représente l'hydrogène, un halogène, les mêmes substituants que $R_3$, un groupe alkylamino en $C_1$–$C_4$ ou di-(alkyle en $C_1$–$C_4$)-amino,

$R_5$ représente l'hydrogène ou un groupe méthyle,

X représente un groupe $-NR_6R_7$ ou 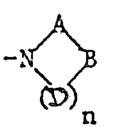

$R_6$ représente un groupe alcényle en $C_3$–$C_6$, alcynyle en $C_3$–$C_6$, cycloalkyle en $C_3$–$C_7$, cycloalcényle en $C_5$–$C_7$, non substitué, ou un groupe alkyle en $C_1$–$C_6$, alcényle en $C_3$–$C_6$, alcynyle en $C_3$–$C_6$, cycloalkyle en $C_3$–$C_7$, substitué par des halogènes, des groupes cyano, -CO-alkyle en $C_1$–$C_5$, -CO-halogénoalkyle en $C_1$–$C_5$, -COOH, -COO-alkyle en $C_1$–$C_5$, COO-halogénoalkyle en $C_1$–$C_5$, -CO-alcoxyalkyle en $C_2$–$C_{10}$, CO-COO-alkyle en $C_1$–$C_5$ ou bien $R_6$ représente un groupe CO-halogénoalkyle en $C_1$–$C_5$, -CO-alcoxyalkyle en $C_2$–$C_{10}$, CO-cycloalkyle en $C_3$–$C_7$ ou -COCOO-alkyle en $C_1$–$C_5$, $R_6$ pouvant également représenter un groupe $-SO_2CH_3$ lorsque $R_7$ représente l'hydrogène,

$R_7$ représente l'hydrogène, un groupe alkyle en $C_1$–$C_4$, les mêmes substituants que $R_6$ ou un groupe $-SO_2$-alkyle en $C_1$–$C_4$,

A représente un groupe -CO-, $-SO_2-$, -COO-, CONH- ou -CON-alkyle en $C_1$–$C_5$,

B représente un groupe alkylène en $C_1$–$C_4$ ou alcénylène en $C_2$–$C_4$,

D représente un groupe -CO-, $-SO_2-$, -CH-alkyle en $C_1$–$C_5$ ou C-(alkyle en $C_1$–$C_5$)$_2$,

n est égal à 0 ou 1,

E représente l'azote =N– ou un groupe méthine =CH–, et

Z représente l'oxygène.

2 Les N-phénylsulfonyl-N′-pyrimidinyl- ou -triazinyl-urées de formule I selon la revendication 1 dans lesquelles

$R_1$ représente l'hydrogène, un halogène ou un groupe alcoxycarbonyle en $C_1$–$C_5$,

$R_2$ représente l'hydrogène,

$R_3$ représente un groupe alkyle en $C_1$–$C_3$, halogénoalkyle en $C_1$–$C_3$, alcoxy en $C_1$–$C_3$ ou halogénoalcoxy en $C_1$–$C_3$,

$R_4$ représente l'hydrogène, un halogène, les mêmes substituants que $R_3$ ou un groupe di-(alkyle en $C_1$–$C_3$)-amino,

$R_5$ représente l'hydrogène ou un groupe méthyle,

X représente un groupe NH-alcényle en $C_3$–$C_5$, NH-cycloalkyle en $C_3$–$C_7$ ou NH-cycloalcényle en $C_5$–$C_7$, non substitué ou substitué par des halogènes, des groupes cyano, CO-halogénoalkyle en $C_1$–$C_5$, CO-alcoxyalkyle en $C_2$–$C_6$ ou COO-alkyle en $C_1$–$C_5$, X peut en outre représenter un groupe NHCHO, NHCO-halogénoalkyle en $C_1$–$C_5$, NHCOO-halogénoalkyle en $C_1$–$C_5$, NHCONH-halogénoalkyle en $C_1$–$C_5$, NHCOCOO-alkyle en $C_1$–$C_5$, $NHSO_2$-alkyle en $C_1$–$C_5$, N-($SO_2$alkyle en $C_1$–$C_5$)$_2$ ou bien X représente un hétérocycle saturé à 5 ou 6 chaînons relié par l'intermédiaire de l'azote et qui peut encore contenir dans le cycle un atome d'oxygène, de soufre ou d'azote et doit contenir au moins un groupe oxo.

3 La N-[2-(2′,2′-dichloropropionylamido)-phénylsulfonyl]-N′-(4-méthoxy-6-méthyl-1,3,5-triazine-2-yl)-urée selon la revendication 1.

4 La N-(2-chloroacétamidophénylsulfonyl)-N′-(4-méthoxy-6-méthyl-1,3,5-triazine-2-yl)-urée selon la revendication 1.

5 La N-(2-méthylsulfonylamido-6-chlorophénylsulfonyl)-N′-(4-méthoxy-6-méthyl-1,3,5-triazine-2-yl)-urée selon la revendication 1.

6 La N-(2-bromacétamidophénylsulfonyl)-N′-(4-méthoxy-6-méthyl-1,3,5-triazine-2-yl)-urée selon la revendication 1.

7 La N-(2-méthoxyacétamidophénylsulfonyl)-N′-(4-méthoxy-6-méthyl-1,3,5-triazine-2-yl)-urée selon la revendication 1.

8 La N-[2-chloracétamido-6-chlorphénylsulfonyl]-N′-(4-méthoxy-6-méthyl-1,3,5-triazine-2-yl)-urée selon la revendication 1.

9 La N-[2-(4′-chlorobutyrylamido)-phénylsulfonyl]-N′-(4-méthoxy-6-méthyl-1,3,5-triazine-2-yl)-urée selon la revendication 1.

10 La N-(2-méthoxyoxalylamidophénylsulfo-

nyl)-N'-(4-méthoxy-6-méthyl-1,3,5-triazine-2-yl)-urée selon la revendication 1.

11 La N-(2-méthoxyoxalylamido-6-chlorophénylsulfonyl)-N'-(4-méthoxy-6-méthyl-1,3,5-triazine-2-yl)-urée selon la revendication 1.

12 La N-(2-chloroacétamidophénylsulfonyl)-N'-(4-méthoxy-6-méthyl-pyrimidine-2-yl)-urée selon la revendication 1.

13 La N-[2-(4'-chlorobutyrylamido)-phénylsulfonyl]-N'-(4-méthoxy-6-méthyl-pyrimidine-2-yl)-urée selon la revendication 1.

14 La N-[2-(2'-pyrrolidone-1'-yl)-phénylsulfonyl]-N'-(4-méthoxy-6-méthyl-pyrimidine-2-yl)-urée selon la revendication 1.

15 Procédé de préparation des N-phénylsulfonyl-N'-pyrimidinyl- ou triazinyl-urées de formule I, revendication 1, caractérisé en ce que l'on fait réagir un benzène-sulfonamide de formule II

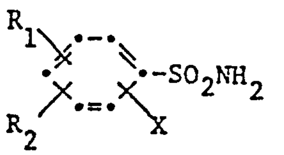

(II)

dans laquelle $R_1$, $R_2$ et X ont les significations indiquées en référence à la formule I, en présence d'une base, avec un N-pyrimidinyl- ou -triazinyl-carbamate de formule

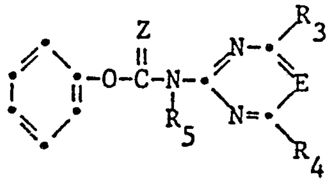

(III)

dans laquelle E, $R_3$, $R_4$, $R_5$ et Z ont les significations indiquées en référence à la formule I, le groupe phényle pouvant éventuellement être substitué, et le cas échéant on convertit en sel.

16 Procédé de préparation des composés de formule I, revendication 1, caractérisé en ce que l'on fait réagir un phénylsulfonyl-isocyanate ou isothiocyanate de formule IV

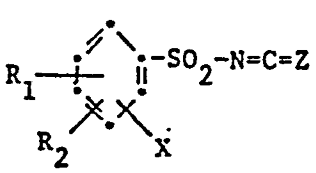

(IV),

dans laquelle $R_1$, $R_2$, X et Z ont les significations indiquées en référence à la formule I, éventuellement en présence d'une base, avec une amine de formule V

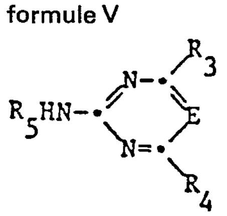

(V),

dans laquelle $R_3$, $R_4$ et $R_5$ ont les significations indiquées en référence à la formule I, et le cas échéant on convertit en sel.

17 Procédé de préparation des composés de formule I, revendication 1, dans laquelle $R_5$ représente l'hydrogène, caractérisé en ce que l'on fait réagir les sulfonamides répondant à la formule II indiquée dans la revendication 15, éventuellement en présence d'une base, avec un isocyanate ou isothiocyanate de formule VI

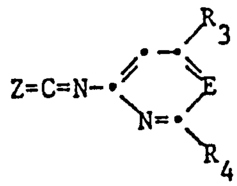

(VI),

dans laquelle E, $R_3$, $R_4$ et Z ont les significations indiquées en référence à la formule I, et le cas échéant on convertit en sel.

18 Procédé de préparation des composés de formule I, revendication 1, caractérisé en ce que l'on fait réagir un N-phénylsulfonylcarbamate de formule VII

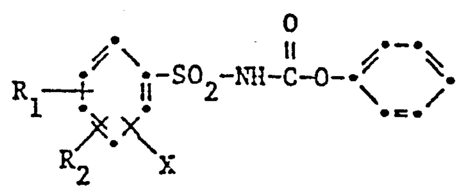

(VII),

dans laquelle $R_1$, $R_2$ et X ont les significations indiquées en référence à la formule I, le groupe phényle pouvant le cas échéant être substitué, avec une amine de formule V ci-dessus et le cas échéant on convertit en sel.

19 Procédé de préparation des sels d'addition de formule I, revendication 1, caractérisé en ce que l'on fait réagir une sulfonylurée de formule I avec une amine, un hydroxyde de métal alcalin ou alcalino-terreux ou une base d'ammonium quaternaire.

20 Phénylsulfonamides de formule II

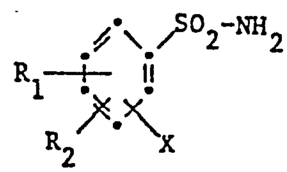

(II),

dans laquelle $R_1$, $R_2$ et X ont les significations indiquées en référence à la formule I, revendication 1.

21 Un produit herbicide et inhibiteur de la croissance des végétaux, caractérisé en ce qu'il contient en tant que substance active, avec des véhicules et/ou d'autres additifs, au moins une N-phénylsulfonyl-N'-triazinyl- ou -pyrimidinyl-urée de formule I, revendication 1.

22 L'utilisation des N-phénylsulfonyl-N'-triazinyl- ou -pyrimidinyl-urées de formule I selon la revendication 1, ou de produits en contenant, pour la lutte sélective en pré-levée ou en post-levée contre les mauvaises herbes dans les cultures de végétaux utiles.

23 L'utilisation des composés de formule I selon la revendication 1 ou de produits en contenant dans les cultures de céréales, de coton, de soja, de maïs et de riz.